# EUROPEAN PATENT APPLICATION

(11) **EP 1 764 094 A1**
(43) Date of publication of application: **21.03.2007**
(21) Application number: 05020511.1
(22) Date of filing: 20.09.2005
(51) Int. Cl.: A61K 31/05, A61P 11/00

(54) **Novel multi-cyclic compounds and their use**

(71) Applicant: Revotar Biopharmaceuticals AG, 16761 Henningsdorf (DE)
(72) Inventor: Kranich, Remo, Dr., 13503 Berlin (DE); Aydt, Ewald M., Dr., 14163 Berlin (DE); Busemann, Anke S., Dr., 16761 Henningsdorf (DE)
(74) Representative: Isenbruck, Günter

(57) **Abstract**

Pharmaceutical compositions comprising at least one compound of the formulas (Ia) or (Ib) or (IIa) or (IIb) and a pharmaceutically acceptable carrier which is useful in a medicine 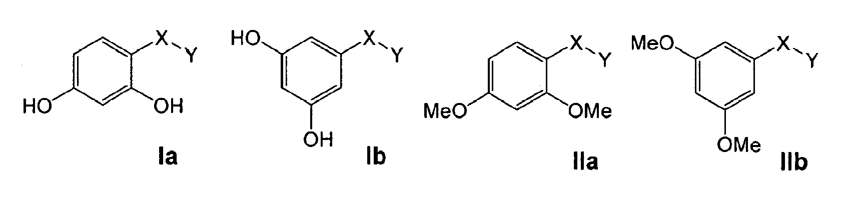 wherein the symbols and substituents have the following meaning
-X- is e.g. 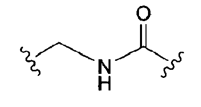 or 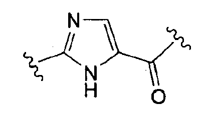 or 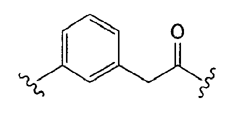 or 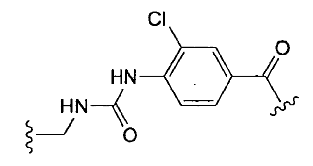 and Y being 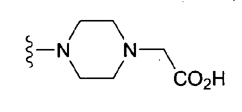 or 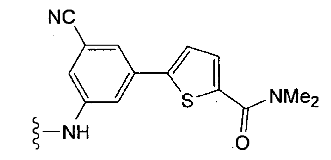 or the pharmaceutically acceptable salts, esters or amides and prodrugs of the above identified compounds of formulas (Ia) or (Ib) or (IIa) or (IIb). The compounds are applied to modulate the in-vitro and in-vivo binding processes mediated by E-, P- or L-selectin binding.

## Description

The present invention relates generally to compounds, compositions and methods for modulating the *in vitro* and *in vivo* processes mediated by cell adhesion molecules. The disclosed small molecules comprise dimethoxy and dihydroxy phenyl subunits and modulate cell adhesion molecule-mediated functions potently.

Cell-adhesion molecule-mediated functions are part of a complex cascade leading to the migration of circulating white blood cells (leukocytes) from the blood stream into the surrounding tissue (transmigration). Physiologically, leukocyte transmigration is of critical importance for homeostasis and immuno-surveillance of living beings including humans. Lymphocytes for example, are constitutively leaving the blood stream into lymphatic tissues in order to patrol for harmful antigens. Under pathological circumstances however, e.g. local or systemic inflammation and/or injury of the vascular system, this fundamental process is dys-regulated, at least in part, due to an increased surface expression of E- and P-selectin. Consequently, the excessive leukocyte transmigration leads to a pathological cellular infiltrate with subsequent tissue damage in several clinically relevant settings. Disease states such as Acute Lung Injury (ALI), Acute Respiratory Distress Syndrome (ARDS), Asthma bronchiale (asthma), Chronic Obstructive Pulmonary Disease (COPD), Psoriasis, Rheumatoid Arthritis, and Sepsis are all associated with tissue inflammation induced and perpetuated by pathologically activated leukocytes infiltrating the respective tissue. In addition, exaggerated leukocyte infiltration contributes to the pathogenesis of Ischemic-Reperfusion Injury (IRI) associated with organ transplantation, cardiopulmonary bypass or percutaneous transluminal angioplasty.

To transmigrate, leukocytes must bind to the wall of the vascular endothelium to diffuse through the cell wall of the capillary into the surrounding tissue. Therefore, leukocytes have to roll onto and then adhere to the endothelial cell wall (initial rolling or "tethering"). This primary event in transmigration is mediated by the selectin family of cell-adhesion molecules. In addition to directly binding to the endothelium, leukocytes can adhere to other leukocytes, leukocyte-particles, platelets or platelet-derived particles that are already attached to the endothelium.

The selectin family of adhesion molecules is comprised of three structurally related calcium-dependent carbohydrate binding cell surface proteins, E-, P- and L-selectin. E-selectin is expressed only on inflamed endothelium, P-selectin is expressed on inflamed endothelium as well as on platelets and L-selectin is expressed on leukocytes. Selectins are composed of an amino terminal lectin domain, an epidermal growth factor (EGF)-like domain, a variable number of complement receptor-related repeats, a hydrophobic transmembrane domain and a C-terminal cytoplasmic domain. The binding interactions leading to the adhesion of the leukocytes are supposed to be mediated by contact of the lectin domain of the selectins and various carbohydrate ligands on the surface of the leukocytes. All three selectins can bind with low affinity to the carbohydrate sialyl Lewis^{x} (sLe^{x}), a glycosyl moiety present on the surface of most leukocytes. A structurally related glycosyl moiety, sialyl Lewis^{a} (sLe^{a}), is predominantly found on the surface of cancer cells [K. Okazaki et al., J. Surg. Res., 1998, 78(1). 78-84; R. P. McEver et al., Glycoconjugate Journal, 1997, 14(5), 585-591]. In case of P-selectin, a distinct high affinity glycoprotein ligand has been described [R.P. McEver, R.D. Cummings, J.Clin.Invest., 1997, 100, 485-492], the so-called P-selectin glycoprotein ligand-1 (PSGL-1), which contributes to a high affinity selectin binding by its sLe^{x} moiety as well as by parts of its peptide components, in particular sulphated tyrosine residues [R.P. McEver, Ernst Schering Res. Found. Workshop, 2004, 44, 137-147]. PSGL-1 is one of the most important selectin ligands binding with highest affinity to P-selectin, but it also binds to E- and L-selectin [G. Constantin; Drug News Perspect; 2004; 17(9); 579-586]. It is a homodimeric sialomucin predominantly expressed on leukocytes.

In inflammatory diseases, dys-regulated transmigration is, at least in part, mediated due to an increased cell surface expression of E- and P-selectin. In contrast to their low basal expression, E- and P-selectin expression is upregulated during inflammation, leading to a substantial recruitment of leukocytes into the inflamed tissue. Although selectin-mediated cell adhesion is required for fighting infection, there are various situations in which such cell adhesion is undesirable or excessive, resulting in severe tissue damage instead of repair. In the case of many acute as well as chronic inflammatory disorders [e.g., asthma, chronic obstructive pulmonary disease (COPD), psoriasis, etc.], an association between infiltration of activated leukocytes into the tissue simultaneously with a marked elevation of tissue expression of corresponding adhesion molecules, particularly E- and P-selectin, has been demonstrated [Muller et al., J. Pathol., 2002, 198(2), 270-275; Di Stefano et al., Am. J Respir. Crit. Care. Med., 1994, 149(3) 803-810; Terajima et al., Arch. Dermatol. Res., 1998, 290, 246-252]

Leukocyte infiltration may also play a role in inflammatory symptoms in the course of transplant and graft rejection. Also the process of blood clotting is further promoted by leukocyte-leukocyte and leukocyte-platelet binding, which occurs because leukocytes possess both L-selectin and its corresponding ligand PSGL-1 and can thus interact with themselves via PSGL-1, and they can also bind to platelets which carry P-selectin.

Therefore, the modulation of selectin-mediated cell adhesion and other selectin mediated functions, e.g. leukocyte activation, offers a promising possibility to interfere with and stop the inflammation cascade at a very early step. Small molecule selectin antagonists should modulate all three selectins simultaneously as pan-selectin-antagonists to circumvent possible redundancies between the selectins [M. Sperandio et al., Vascular Disease Prevention, 2004, 1, 185-195].

Besides sLe^{x}/sLe^{a}, the natural, high affinity ligand PSGL-1 is another template structure for the design of small molecule selectin antagonists. As compared to sLe^{x}/sLe^{a}, PSGL-1 shows high affinity for all three selectins. To find and to detect novel small molecule drugs that compete with PSGL-1 and PSGL-1-like ligands for selectin binding is therefore a promising strategy to develop a novel class of effective pan-selectin antagonists for treating inflammatory disorders. Selectin antagonists may be designed using selectins as well as using a ligand like PSGL-1 as a template structure, since they are intended to modulate the binding between selectins and PSGL-1 or other ligands with similar binding motifs.

Novel small molecule selectin antagonists could meet certain requirements to be drug-like and to have potential oral bioavailability. The term drug likeness is described in the literature [Lipinski; Adv. Drug Dev. Rev., 1997, 23, 3-25]. Beside other molecular properties, passively transported molecules are supposed to have on average a relative molecular weight of less than 500 in order to be drug like. According to these rules it is common to define compounds with a relative molecular weight of less 500 or closely above that as small molecules. Compounds with relative molecular weights above 500 are unlikely to be orally bioavailable. Also the presence of highly polar carbohydrate moieties or a peptidic components is not in accordance with the concept of drug likeness [H. Ulbrich et al., Trends Pharmacol. Sci., 2003, 24(12), 640-647; D. Slee et al., J. Med. Chem., 2001, 44, 2094-2107]. The same accounts for the development of antibody-based drugs, because they are polypeptides and so oral administration is a problem. Moreover, the desired compounds must be stable during the passage through the gastrointestinal tract so that they can be ingested/absorbed latest by the cells of the small intestines. This is not the case for most glycosidic molecules and peptidic structures.

There have been various investigations to develop low-molecular weight compounds with a modulatory effect on selectin mediated processes. These compounds include disalicylates and disalicylate-based C-glycosides [WO 99/29706], benzyl amino sulfonic acids [WO 03/097658], diglycosylated 1,2-diols [WO 97/01569], substituted 5-membered heterocycles [WO 00/33836], mannopyranosyloxy-phenyl-benzoic acids [EP0758243 B1], piperazine based compounds [US6432957B1], gallic acid derivatives of peptides [WO 2004/018502], gallic acid [C. C. M. Appeldoorn et al., Circulation 2005, 111, 106-112; EP 1481669A1], and quinic acid derivatives [N. Kaila et al., J. Med. Chem. 2005, 48, 4346-4357]. However, none of these selectin-antagonizing compounds have successfully passed clinical trials up to date [S. J. Romano, Treat. Respir Med 2005, 4(2), 85-94; M. P. Schön, Therapeutics and Clinical Risk Management, 2005, 1(3), 201-208]. This is due to the fact, that many of these structures have been designed on the basis of the low potency template sLe^{x}. Therefore, sLe^{x}-mimicking structures are unlikely to show low potency. Other compounds show specificity against different members of the selectin family, but antagonizing only selected selectins can be bypassed by other selectins [M. P. Schön, Therapeutics and Clinical Risk Management, 2005, 1(3), 201-208]. In addition, most of the compounds developed so far have high molecular weights and often bear carbohydrates and/or peptides making them prone to degradation and modification by peptidases and/or glycosidases. Carbohydrate-bearing structures have further disadvantages such as high degree of chirality, anomericity, and low probability of transport through lipid bilayers. Similar disadvantages are known for peptide-bearing compounds. Some other compounds developed for antagonizing selectin mediated processes contain pyrogallol- and catechol-substructures. These motifs are prone to oxidation processes [Kumamoto M. et al., Biosci. Biotechnol. Biochem., 2001, 65(1), 126-132] making the pharmaceutical development of these compounds difficult. In addition, compounds with pyrogallol substructures, such as gallic acid, are known to be cytotoxic [E. Sergediene et al., FEBS Letters, 1999, 462, 392-396] and induce apoptosis [K. Satoh et al., Anticancer Research, 1997, 17, 2487-2490; N. Sakaguchi et al., Biochemical Pharmacology, 1998, 55, 1973-1981].
The leading compound in the field of selectin antagonists is bimosiamose [S. J. Romano, Treat. Respir Med 2005, 4(2), 85-94]. Presently bimosiamose [D. Bock et al., *New Drugs*, 2003, D04, 28, p.28; EP 0 840 606 B1] is the most advanced compound in clinical studies Recent investigations support the hypothesis that bimosiamose can be considered as PSGL-1 mimetic [E. Aydt, G. Wolff; Pathobiology; 2002-2003; 70; 297-301]. This distinguishes bimosiamose from other selectin antagonists. It is, however, a high molecular weight compound with carbohydrate structures. The pan-selectin antagonist bimosiamose seems to lack oral bioavailability. Some observations indicate that bimosiamose shows good affinity for P-selectin and a moderate affinity for E- and L-selectin.

There is a strong medical need for novel highly potent pan-selectin antagonists which modulate selectin-mediated function, e.g. of selectin-dependent cell adhesion, and for the development of methods employing such compounds to modulate conditions associated with selectin-ligand interaction. Most of the available anti-inflammatory pharmaceutical therapies, which are available on the market, comprise mostly corticosteroids or NSAIDs (non steroidal anti-inflammatory drugs) having several serious drawbacks/side effects, and target different steps of the inflammatory cascade. Unlike this, modulating the selectin function is a therapeutic concept intervening the inflammation cascade at a very early stage. Almost all promising selectin antagonists so far failed to become marketed drugs, mostly because of low potency and /or high molecular weight that causes problems in their absorption-distribution-metabolism-excretion (ADME) behaviour and thus in oral bioavailability required for the treatment of most inflammatory disorders like rheumatoid arthritis, septic shock, atherosclerosis, reperfusion injury and many others.

Object of the invention is to provide novel small molecules, especially non-glycosylated/non-glycosidic and non-peptidic compounds, which are able to potently antagonize selectin-mediated processes and which have less negative side effects during their application than prior art compounds.

Unlike most of the sLe^{x}-mimicking compounds developed in this field, the inventive compounds are not prone to glycosidases or peptidases. Most of the selectin antagonists developed so far are structurally and biologically based on the properties of sLe^{x} or sLe^{a}. These resulting compounds showed, therefore, low biological activity like their template structures. This invention, however, provides novel potent small and drug like pan-selectin antagonists that have been invented on the basis of biological in vitro assays mimicking PSGL-1 and PSGL-1-like ligands or any ligands bearing sLe^{x} or sLe^{a} and tyrosinesulfate motifs [N. V. Bovin; Biochem Soc Symp.; 2002;(69):143-60. N. V. Bovin; Glycoconj. J.; 1998; 15(5); 431-46. T.V. Pochechueva et al.; Bioorg Med Chem Lett.; 2003;13(10);1709-12. G. Weitz-Schmidt et al.; Anal. Biochem.; 1996; 238; 184-190].

The present invention provides pharmaceutical compositions comprising at least one compound having the general structure of formulas (Ia) or (Ib) or (IIa) or (IIb) and a pharmaceutically acceptable carrier which is useful in medicine. 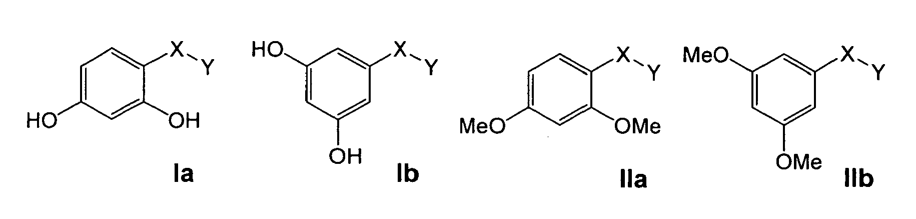 wherein the symbols and substituents have the following meaning
-X-= 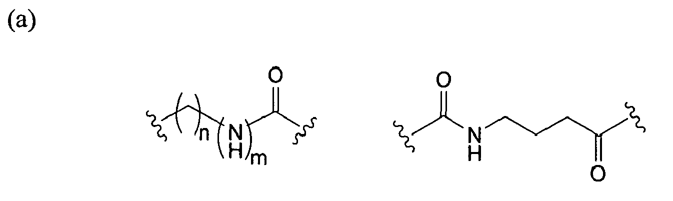 with m = 0,1; n = an integer from 1 to 3 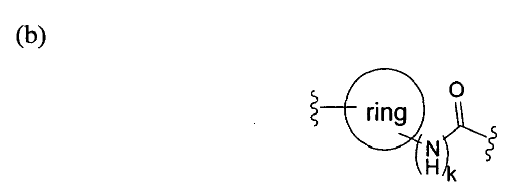 wherein "ring" is 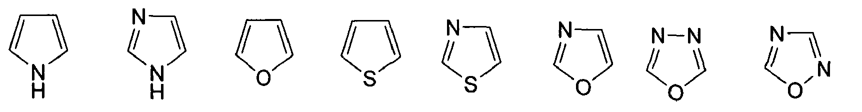 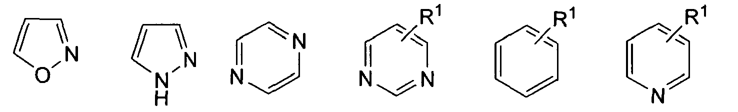 and with R¹ being H, NO₂, CF₃, F, Cl, Br, I, CN, CH₃, NH₂, NHAlkyl, NHAryl, NHAcyl and k = 0,1 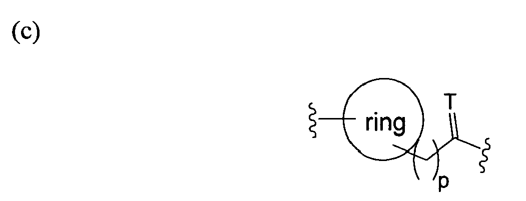 T being O, S or [H,H]; p = 0,1,2, 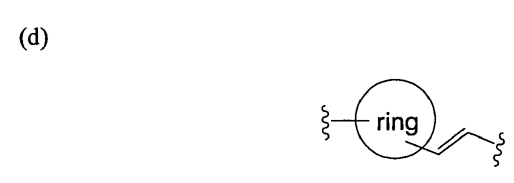 the double bond is either *E*- or *Z*-configurated 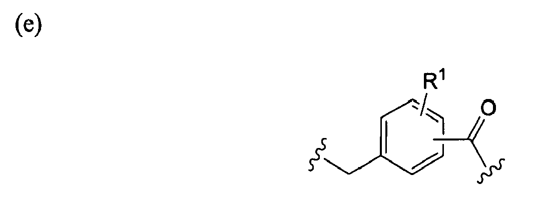 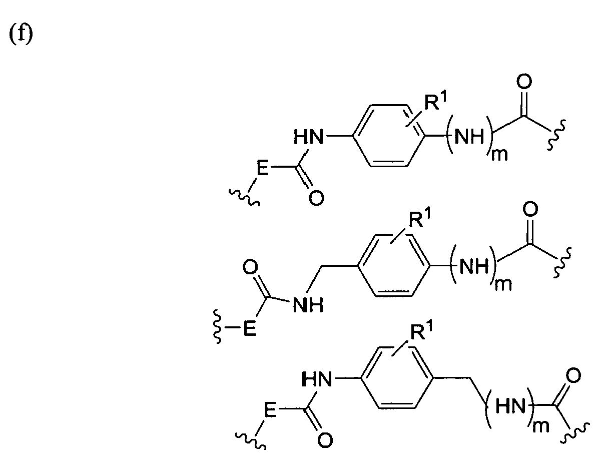 with -E- being -(CH₂-)_{q}NH- and q = 0, 1, 2, 3
-Y = 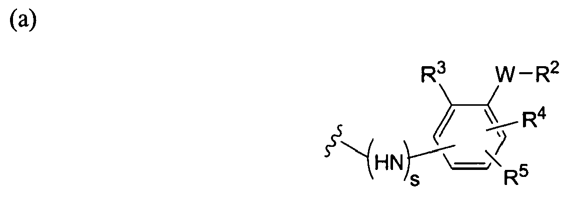 with
   s being 0 or 1,
   R² being CO₂H, CO₂Alkyl, CO₂Aryl, CO₂NH₂, CO₂Aralkyl, SO₃H, SO₂NH₂, PO(OH)₂, 1-H-tetrazolyl-, CHO, COCH₃, CH₂OH, NH₂, NHAlkyl, N(Alkyl)Alkyl', OCH₃, CH₂OCH₃, SH, F, Cl, Br, I, CH₃, CH₂CH₃, CN, CF₃
   R³ independently from R² being H, CH₃, CH₂CH₃, CF₃, F, Cl, Br, I, CN, NO₂ and
   R⁴ independently from R² and R³ being H, CH₃, CH₂CH₃, CF₃, F, Cl, Br, I, CN, NO₂, R²
   R⁵ being H, NO₂, CF₃, F, Cl. Br, I, CN, CH₃, OCH₃, SH, NH₂
   and -W- = -(CH₂-)ᵥ, cis-CH=CH- or *trans*-CH=CH-, and v being 0,1,2;
   in case that -W- is cis-CH=CH- or *trans*-CH=CH-, R² must not be NH₂ or SH; 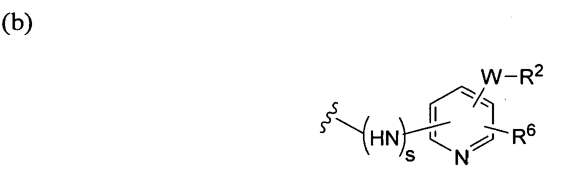
   R⁶ independently from R² being H, F, Cl, Me, tert-Bu, CN, NH₂ 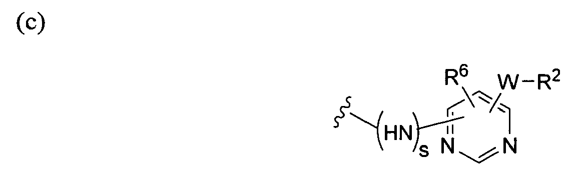 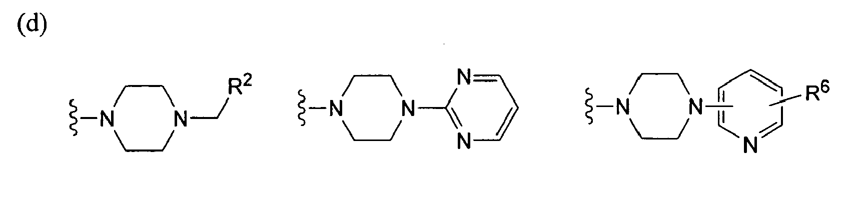 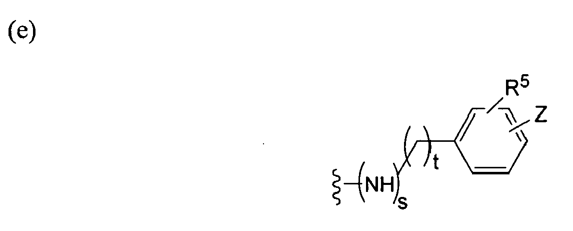 with t being 0,1,2 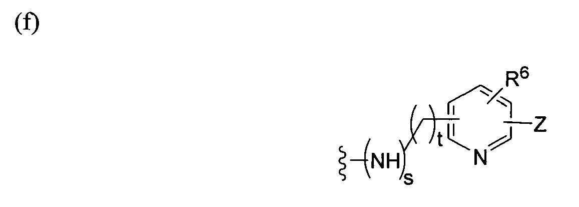 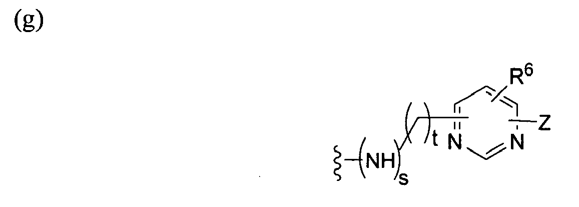
-Z = 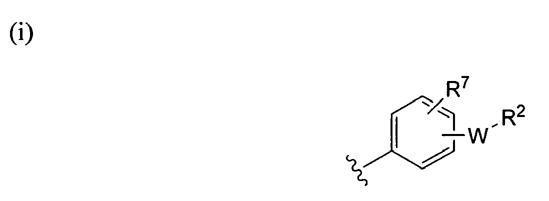 R⁷ independently from R² being H, NO₂, CF₃, F, Cl. Br, I, CN, CH₃, OCH₃, SH, NH₂, 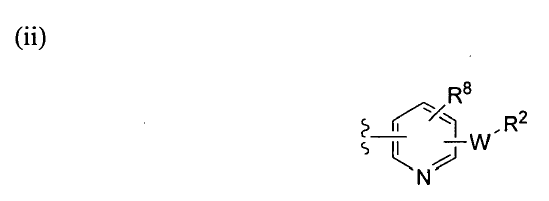 R⁸ independently from R² being H, F, Cl, Me, tert-Bu, CN, NH₂ 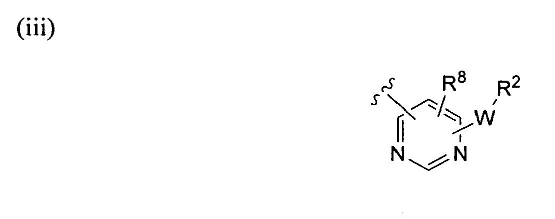 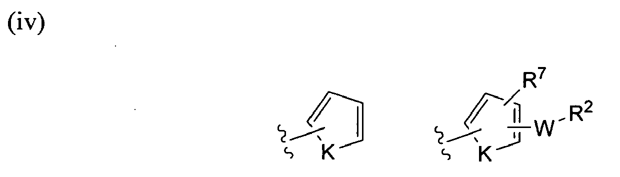 with K = NH, NMe, O, S

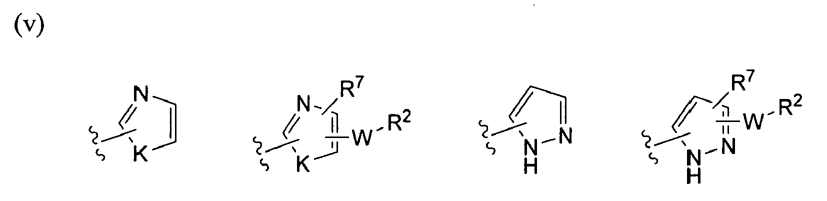

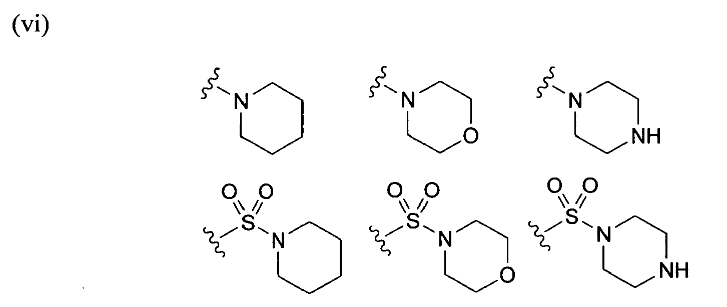

   (vii) -W-R²
or the pharmaceutically acceptable salts, esters or amides and prodrugs of the above identified compounds of formulas (Ia) or (Ib) or (IIa) or (IIb).

Preferred pharmaceutical compositions comprise compounds of formulas (IIIa) or (IIIb) or (IVa) or (IVb) 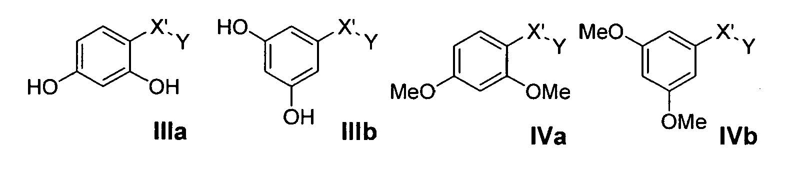 wherein -Y is like defined above and wherein -X'- is X (a), X (b), X (c), and X (d) like defined above.

Further preferred pharmaceutical compositions comprise compounds of formulas (A1), (A2), (B1), (B2), (C1), (C2), (D1), or (D2) 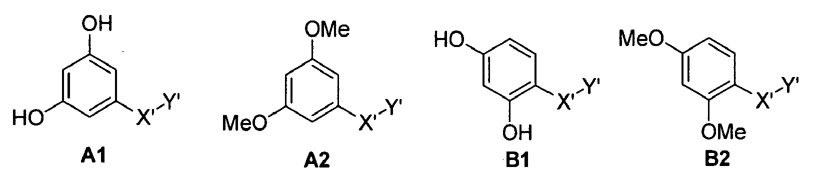 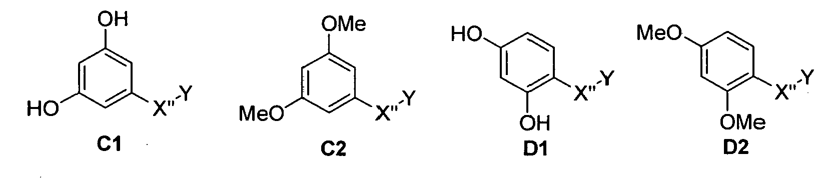 wherein -X'- and -Y are like defined above and wherein -X"- is 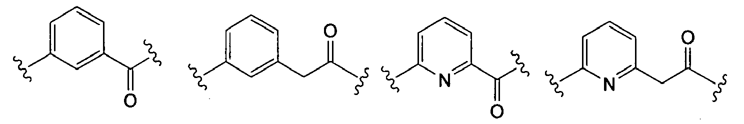 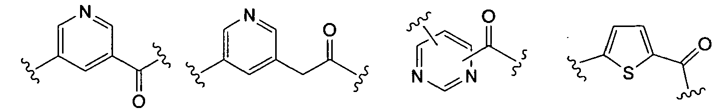 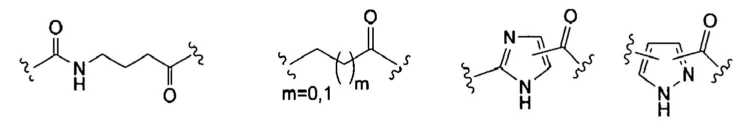 and wherein -Y' is 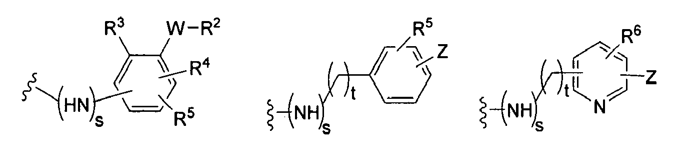 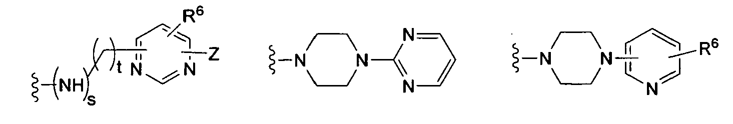 wherein all indices, symbols and substituents are like defined above

Particularly preferred pharmaceutical compositions comprise compounds of formulas (E1), (E2), (F1), or (F2) 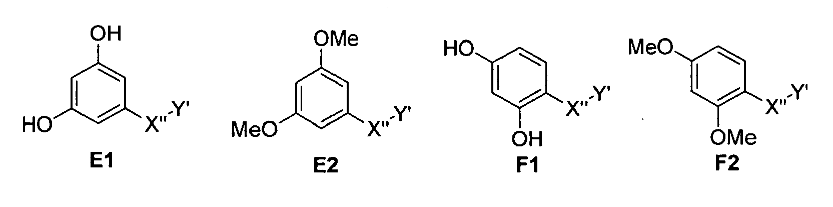 wherein -X"- and -Y' are like defined above.

Very particularly preferred pharmaceutical compositions comprise compounds of formulas (G1), (G2), (H1), or (H2) 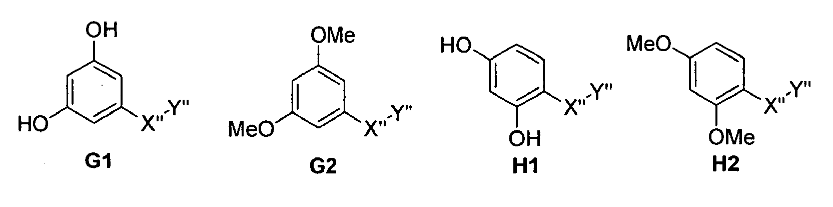 wherein -X"- is like defined above and -Y" is 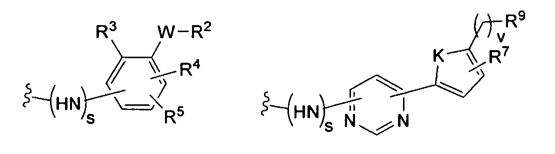 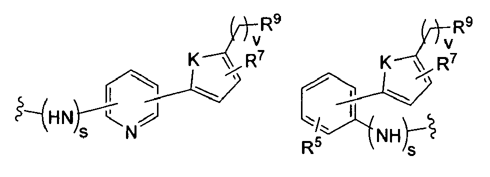 with R⁹ being CO₂H, CO₂alkyl, CO₂aryl, CO₂NH₂, CO₂aralkyl, CH₂SO₃H, CH₂SO₂NH₂, CH₂PO(OH)₂, 1-H-tetrazolyl, CHO, COCH₃, CH₂OH, CH₂NH₂, CH₂NHalkyl, CH₂N(alkyl)alkyl', CH₂OCH₃, CH₂SH

These chemical compounds (E1), (E2), (F1), (F2), (G1), (G2), (H1) and (H2) are also new compounds for themselves.

All compounds as described before present the ability of modulating cell adhesion and modulate selectin- as well as PSGL-1-like mediated binding. The compounds have the ability to modulate the interaction of selectins with sLe^{x}/sLe^{a} and also the interaction between selectins and tyrosinesulfate residues. Therefore they are useful for the treatment of acute and chronic inflammatory disorders, as well as other medical conditions where selectin mediated processes play a role.

The term "pharmaceutical" includes also diagnostic applications.
The term "pharmaceutical" includes also prophylactic applications in order to prevent medical conditions where selectin mediated processes play a role.
The term "pharmaceutical" includes also applications, where compounds of the present invention may be used as vehicles for drug targeting of diagnostics or therapeutics.

In a further preferred variant the invention provides pharmaceutical compositions comprising at least one compound of formula (A1), (A2), (B1), (B2), (C1), (C2), (D1), (D2), (E1), (E2), (F1), (F2), (G1), (G2), (H1), or (H2).
The invention provides pharmaceutical compositions comprising compounds of formulas (Ia) or (Ib) or (IIa) or (IIb) and in a preferred variant of formulas (IIIa) or (IIIb) or (IVa) or (IVb).
In a further preferred variant the invention provides pharmaceutical compositions comprising at least one compound of formula (A1), (A2), (B1), (B2), (C1), (C2), (D1) or (D2).
In a particularly preferred variant the invention provides pharmaceutical compositions comprising at least one compound of formula (E1), (E2), (F1) or (F2).
In a very particularly preferred variant the invention provides pharmaceutical compositions comprising at least one compound of formula (G1), (G2), (H1) or (H2).

The present invention further provides a method of nodulating the binding of P-selectin, L-selectin or E-selectin to sLe^{x} or sle^{a} and tyrosinesulfate residues comprising the step of administering to a patient an effective amount of at least one compound having the structure of formulas (Ia) or (Ib) or (IIa) or (IIb) to modulate the binding of P-, E- or L-selectin to sLe^{x} or sLe^{a} and tyrosinesulfate. It has been found that compounds having the formulas (Ia) or (Ib) or (IIa) or (IIb) shown above act to modulate E-, P- or L-selectin binding.

As used herein the terms "alkyl" and "alkyl'" shall mean a monovalent straight chain or branched chain group of 1 or 2 or 3 or 4 or 5 or 6 or 7 or 8 or 9 or 10 or 11 or 12 carbon atoms including, but not limited to, methyl, ethyl, n-propyl, isopropyl, n-butyl, sec-butyl, tert-butyl and the like. "Alkyl" and "alkyl'" are independently from each other and can be different or identical.

The term "aryl" shall mean carbocyclic and heterocyclic aromatic groups including, but not limited to, phenyl, 1-naphthyl, 2-naphthyl, fluorenyl, (1,2)-dihydronaphthyl, indenyl, indanyl, thienyl, benzothienyl, thienopyridyl and the like.

The term "aralkyl" (also called arylalkyl) shall mean an aryl group appended to an alkyl group including, but not limited to, benzyl, 1-naphthylmethyl, 2-naphthylmethyl, fluorobenzyl, chlorobenzyl, bromobenzyl, iodobenzyl, alkoxybenzyl (wherein "alkoxy" means methoxy, ethoxy, isopropoxy, n-butoxy, sec-butoxy, tert-butoxy an the like), hydroxybenzyl, aminobenzyl, nitrobenzyl, guanidinobenzyl, fluorenylmethyl, phenylmethyl(benzyl), 1-phenylethyl, 2-phenylethyl, 1-naphthylethyl and the like.
The term "acyl" shall mean -(CHO) or -(C=O)-alkyl or -(C=O)-aryl or -(C=O)-aralkyl including, but not limited to, formyl, acetyl, n-propionyl, isopropionyl, n-butyryl, isobutyryl, pivaloyl, benzoyl, 4-nitrobenzoyl and the like.

The term "pharmaceutically acceptable salts, esters, amides and prodrugs" as used herein refers to those carboxylate salts, amino acid addition salts, esters, amides and prodrugs of the compounds of the present invention which are, within the scope of sound medical judgement, suitable for use in contact with tissues of patients without undue toxicity, irritation, allergic response and the like, commensurate with a reasonable benefit/risk ratio, and effective for their intended use, as well as the zwitterionic forms, where possible, of the compounds of the present invention. The term "salts" refers to the relatively non-toxic, inorganic and organic acid addition salts of the compounds of the present invention. These salts can be prepared *in situ* during the final isolation and purification of the compounds or by separately reacting the purified compounds in its free form with a suitable inorganic or organic acid or base and isolating the salt thus formed. Representative salts of the compounds of the present invention include the hydrobromide, hydrochloride, sulfate, bisulfate, nitrate, acetate, oxalate, valerate, palmitate, stearate, laurate, borate, benzoate, lactate, phosphate, tosylate, citrate, maleate, fumarate, succinate, tartrate, naphthylate, mesylate, glucoheptonate, lactiobionate, laurylsulphonate salts and the like. These may include cations based on the alkali and alkalineearth metals, such as sodium, lithium, potassium, calcium, magnesium and the like, as well as non-toxic ammonium, quaternary ammonium and amine cations including, but not limited to, ammonium, tetramethylammonium, tetraethylammonium, methylamine, dimethylamine, trimethylamine, triethylamine, ethylamine, and the like.

Examples of the pharmaceutically acceptable, non-toxic esters of the compounds of this invention include C₁, C₂, C₃, C₄, C₅ and C₆ alkyl esters wherein the alkyl group is a straight or branched chain. Acceptable esters also include C₅, C₆ and C₇ cycloalkyl esters as well arylalkyl esters such as, but not limited to benzyl. C₁, C₂, C₃, C₄, C₅ and C₆ alkyl ester are preferred. Esters of the compounds of the present invention may be prepared according to conventional methods.

Examples of pharmaceutically acceptable, non-toxic amides of compounds of this invention include amides derived from ammonia, primary C₁, C₂, C₃, C₄, C₅ and C₆ alkyl amines and secondary C₁, C₂, C₃, C₄, C₅ and C₆ dialkyl amines wherein the alkyl groups are straight or branched chains. In the case of secondary amines the amine may also be in the form of a 5 or 6 membered heterocycle containing one nitrogen atom. Amides derived from ammonia, C₁, C₂ and C₃ alkyl primary amides and C₁ to C₂ dialkyl secondary amides are preferred. Amides of the compounds of the present invention may be prepared according to conventional methods.

The term "prodrug" refers to one or more compounds that are rapidly transformed *in vitro* and from a non-active to active state *in vivo* to yield the parent compound of the above formulas (Ia) or (Ib) or (IIa) or (IIb), for example by hydrolysis in blood or *in vivo* metabolism.

It is also contemplated that pharmaceutically active compositions may contain a compound of the present invention or other compounds that modulate or compete with E-selectin or P-selectin or L-selectin binding.

Pharmaceutically active compositions of the present invention comprise a pharmaceutically acceptable carrier and a compound of formulas (Ia) or (Ib) or (IIa) or (IIb), whereby a pharmaceutically acceptable carrier can also be a medically appropriate nano-particle, dendrimer, liposome, microbubble or polyethylene glycol (PEG). The pharmaceutical compositions of the present invention may include one or more of the compounds having the above structure (Ia) or (Ib) or (IIa) or (IIb) formulated together with one or more, physiologically acceptable carriers, adjuvants or vehicles, which are collectively referred to herein as carriers, for parenteral injection, for oral administration in solid or liquid form, for rectal or topical administration and the like.

The compositions can be administered to humans and animals either orally, rectally, parenterally (intravenously, intramuscularly, intradermaly or subcutaneously), intracisternally, intravaginally, interperitoneally, locally (powders, ointments or drops), or as a buccal or by inhalation (nebulized, or as nasal sprays).

Compositions suitable for parenteral injection may comprise physiologically acceptable sterile aqueous or nonaqueous solutions, stabilizers, antioxidants, preservatives (e.g. ascorbic acid, sodium sulfite, sodium hydrogene sulfite, benzyl alcohol, EDTA), dispersions, suspensions or emulsions and sterile powders for reconstitution into sterile injectable solution or dispersion. Examples of suitable aqueous and nonaqueous carriers, diluents, solvents or vehicles include water, ethanol, polyol, (propylene glycol, polyethylene glycol, glycerol and the like), suitable mixtures thereof, vegetable oils (such as olive or canola oil) and injectable organic esters such as ethyl oleate. Proper fluidity can be maintained, for examples, by the use of a coating such as lecithin, by the maintenance of the required particle size in the case of dispersions and by the use of surfactants.

These compositions may also contain adjuvants such as preserving, wetting, emulsifying, and dispersing agents. Prevention of the actions of microorganisms can be ensured by various antibacterial and antifungal agents, for examples, parabens, chlorobutanol, phenol, sorbic acid, and the like. It may also be desirable to include isotonic agents, for examples sugars, sodium chloride and the like. Prolonged absorption of the injectable pharmaceutical form can be brought about by the use of agents delaying absorption, for examples aluminium monostearate and gelatin.

If desired, and for more effective distribution, the compounds can be incorporated into slow or timed release or targeted delivery systems such as polymer matrices, liposomes, and microspheres. They may be sterilized, for example, by filtration through a bacteria-retaining filter, or by incorporating sterilizing agents in the form of sterile water, or some other sterile injectable medium immediately before use.

Solid dosage forms for oral administration include capsules, tablets, pills, powders and granules. In such solid dosage forms, the active compound or a prodrug is admixed with at least one inert customary excipient (or carrier) such as sodium citrate or dicalcium phosphate or (i) fillers or extenders, as for example, starches, lactose, sucrose, glucose, mannitol and silicic acid, (ii) binders, as for example, carboxymethylcellulose, alginates, gelatine, polyvinylpyrrolidone, sucrose and acacia, (iii) humectants, as for example, glycerol, (div disintegrating agents, as for example, agar-agar, calcium carbonate, potato or tapioca starch, aliginic acid, certain complex silicates and sodium carbonate, (v) solution retarders, as for examples, paraffin, (vi) absorption accelerators, as for example, quaternary ammonium compounds, (vii) wetting agents, as for examples, cetyl alcohol and glycerol monostearate, (viii) adsorbents, as for example, kaolin and bentonite, and (ix) lubricants, as for example, talc, calcium stearate, magnesium stearate, solid polyethylene glycols, sodium lauryl sulfate and mixtures thereof. In the case of capsules, tablets, and pills, the dosage forms may also comprise buffering agents.

Solid compositions of a similar type may also be employed as fillers in soft and hard-filled gelatine capsules using excipients as lactose or milk sugars as well as high molecular polyethylene glycols and the like. Solid dosage forms such as tablets, dragées, capsules, pills and granules can be prepared with coatings and shells, such as enteric coatings and others well known in the art. They may contain opacifying agents, and can also be of such compositions that they release the active compound or compounds in a certain part of the intestinal tract in a delayed manner. Examples of embedding compositions that can be used are polymeric substances and waxes. The active compounds can also be in microencapsulated form, if appropriate, with one or more of the above-mentioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art such as water or other solvents, solubilizing agents and emulsifiers, as for example, ethyl alcohol, isopropyl alcohol, ethyl carbonate, ethyl acetate, benzyl alcohol, benzyl benzoate, propylene glycol, 1,3-butylene glycol, dimethylformamide, oils, in particular, cottonseed oil, groundnut oil, corn germ oil, olive oil, cannola oil, caster oil and sesame seed oil, glycerol, tetrahydrofurfuryl alcohol, polyethylene glycols and fatty acid esters of sorbitan or mixtures of these substances, and the like. Besides such inert diluents, the compositions can also include adjuvants, such as wetting agents, emulsifying and suspending agents, sweeting, flavouring and perfuming agents.

Suspensions, in addition to the active compounds, may contain suspending agents, for example, ethoxylated isostearyl alcohols, polyoxyethylene sorbitol and sorbitan esters, microcrystalline cellulose, aluminium metahydroxide, bentonite, agar-agar, tragacanth or mixtures of these substances and the like.

Compositions for rectal administrations are preferably suppositories, which can be prepared by mixing the compounds of the present invention with suitable nonirritating excipients or carriers such as cacao butter, polyethylene glycol or a suppository wax, which are solid at ordinary temperatures but liquid at body temperature and therefore melt in the rectal or vaginal cavity and release the active component. Dosage forms for topical administration of a compound of this invention include ointments, powder, sprays and inhalants.

The active component is admixed under sterile conditions with a physiologically acceptable carrier and any needed preservatives, buffers or propellants as may be required. Ophthalmic formulations, eye ointments, suspensions, powder and solutions are also contemplated as being within the scope of this invention.

The compounds of the present invention can also be incorporated into or connected to liposomes or administrated in the form of liposomes. As is known in the art, liposomes are generally derived from phospholipids or other lipid substances. Liposomes are formed by mono or multilamellar hydrated liquid crystals that are dispersed in an aqueous medium. Any non-toxic, physiologically acceptable metabolized lipid capable of forming liposomes can be used. The present compositions in liposome form can contain, in addition to the selectin binding antagonists of the present invention, stabilizers, preservatives, excipients and the like. The preferred lipids are the phospholipids and the phosphatidyl cholines (lecithins), both natural and synthetic. Methods to form liposomes are well known in the art.

Non-parenteral dosage forms may also contain a bioavailability enhancing agent (e.g. enzyme modulateors, antioxidants) appropriate for the protection of the compounds against degradation. Actual dosage levels of active ingredient in the composition of the present invention may be varied so as to obtain an amount of active ingredient that is effective to obtain the desired therapeutic response for a particular composition and method of administration. The selected dosage level, therefore, depends on the desired therapeutic effect, on the route of administration, on the desired duration of treatment and other factors. The total daily dosage of the compounds on this invention administered to a host in single or divided doses may be in the range up to 50 mg per kilogram of body weight. Dosage unit compositions may contain such submultiples thereof as may be used to make up the daily dosage. It will be understood, however, that the specific dose level for any particular patient, whether human or other animal, will depend upon a variety of factors including the body weight, general health, sex diet, time and route of administration, rates of absorption and excretion, combination with other drugs and the severity of the particular disease being treated.

In particular, the compounds of the present invention may be used to treat a variety of diseases relating to inflammation and cell-cell recognition and adhesion. For example, the compounds of the present invention may be administrated to a patient to treat Chronic Obstructive Pulmonary Disease (COPD), acute lung injury (ALI), cardiopulmonary bypass, acute respiratory distress syndrome (ARDS), Crohn's disease, septic shock, sepsis, chronic inflammatory diseases such as psoriasis, atopic dermatitis, and rheumatoid arthritis, and reperfusion injury that occurs following heart attacks, strokes, atherosclerosis, and organ transplants, traumatic shock, multi-organ failure, autoimmune diseases like multiple sclerosis, percutaneous transluminal angioplasty, asthma and inflammatory bowel disease. In each case, an effective amount of the compounds of the present invention is administered either alone or as part of a pharmaceutically active composition to a patient in need of such treatment. It is also recognized that a combination of the compounds may be administered to a patient in need of such administration. The compounds of the present invention may also be administered to treat other diseases that are associated with cell-cell adhesion. As the present compounds modulate the binding of E-selectin or P-selectin or L-selectin, any disease that is related to this interaction may potentially be treated by the modulation of this binding interaction.

In addition to being found on some white blood cells, sLe^{a} is found on various cancer cells, including lung and colon cancer cells. It has been suggested that cell adhesion involving sLe^{a} may be involved in the metastasis of certain cancers and antagonists of sLe^{a} binding might be useful in treatment of some forms of cancer.

Many of the compounds of the present invention may be synthesized according to the following general synthetic schemes.

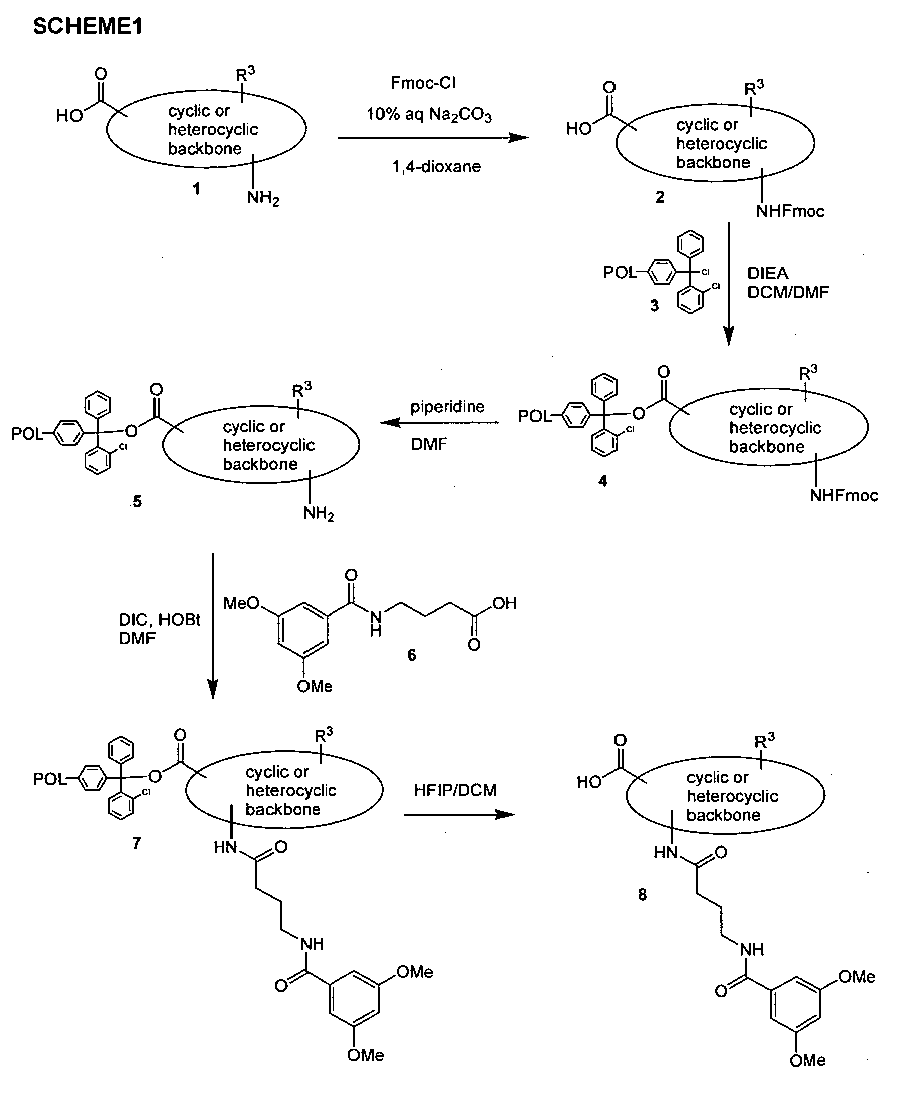

In SCHEME 1 an amino acid of type (1) is reacted with Fmoc-Cl in dioxane under basic conditions (10% Na₂CO₃ in water) to the corresponding *N*-Fmoc protected acid (2).

Carboxylic acid (2) is immobilized to a 2-chlorotrityl chloride resin (3) to form the solid phase supported 2-chlorotrityl ester (4). Deprotection of (4) with piperidine in DMF gives amine of type (5). Further reaction of amine (5) with carboxylic acid (6) under standard coupling conditions (DIC and HOBt in DMF) gives amide (7) which is easily released from the resin with Hexafluoroisopropanol (HFIP) in dichloromethane to obtain carboxylic acids of type (8). Alternatively N'-(3-dimethylaminopropyl)-N-ethyl carbodiimide (EDC), triethylamine and 4-dimethylaminopyridine (DMAP) in a chlorinated solvent may be used for the amide coupling reaction step. The synthesis sequence shown in SCHEME 1 leading to compounds like (8) is not only reduced to the Y-H building blocks like (1) but may be generally applied to all other Y-H type building blocks bearing a carboxylic and a NH₂-function.

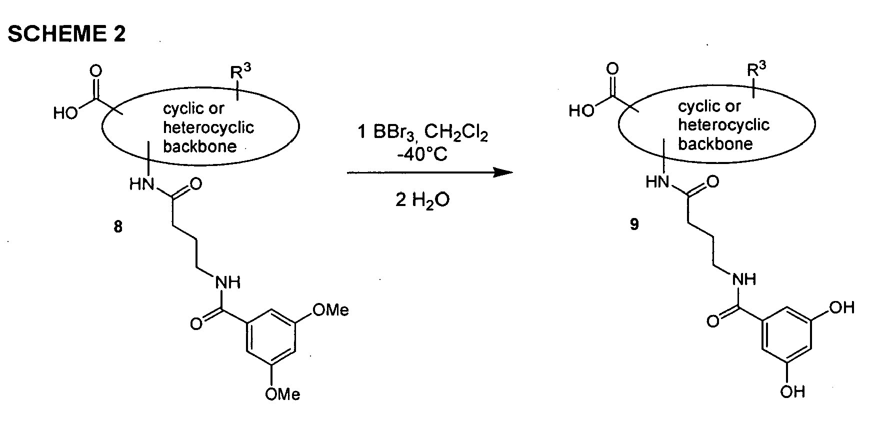

In SCHEME 2 carboxylic acids of type (8) are reacted with boron tribromide in dichloromethane at -40°C to obtain after following aqueous workup corresponding demethylated acids of type (9). The synthesis sequence shown in SCHEME 2 leading to compounds like (9) is not only reduced to X-Y-H and Y-H building blocks like (8) but may be generally applied to all other X-Y-H and Y-H type building blocks.

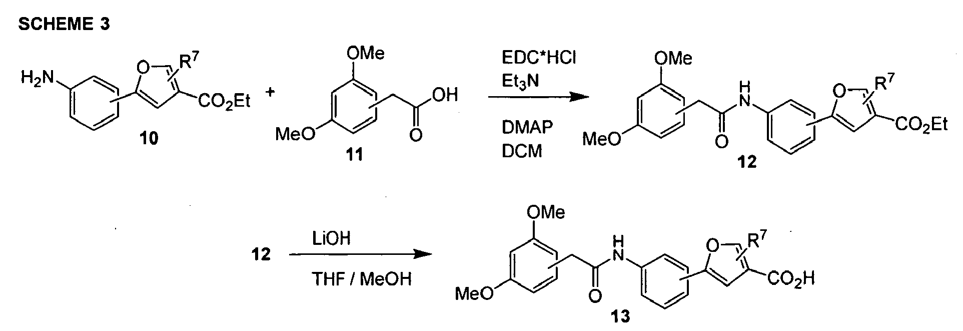

In SCHEME 3 an aniline of type (10) is reacted under inert atmosphere conditions with N'-(3-dimethylaminopropyl)-N-ethyl carbodiimide (EDC), triethylamine, 4-dimethylamino-pyridine (DMAP) and carboxylic acid of type (11) in dichloromethane to give an amide of type (12). Further hydrolysis of ester (12) with aqueous LiOH in THF and MeOH leads to carboxylic acids of type (13).

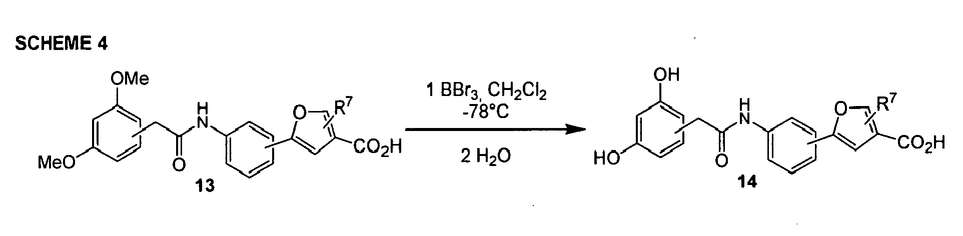

In SCHEME 4 carboxylic acids of type (13) are reacted with boron tribromide in dichloromethane at -40°C to obtain after following aqueous workup corresponding demethylated acids of type (14). The synthesis sequence shown in SCHEME 4 leading to compounds like (14) is not only reduced to X-Y-H and Y-H building blocks like (13) but may be generally applied to all other X-Y-H and Y-H type building blocks.

The present invention is furthermore illustrated by the following representative examples.

### EXAMPLE 1

### 3-[4-(3,5-Dihydroxy-benzoylamino)-butyrylamino]-benzoic acid (21) and 3-[4-(3,5-Dihydroxy-benzoylamino)-butyrylamino]-benzoic acid methyl ester (22)

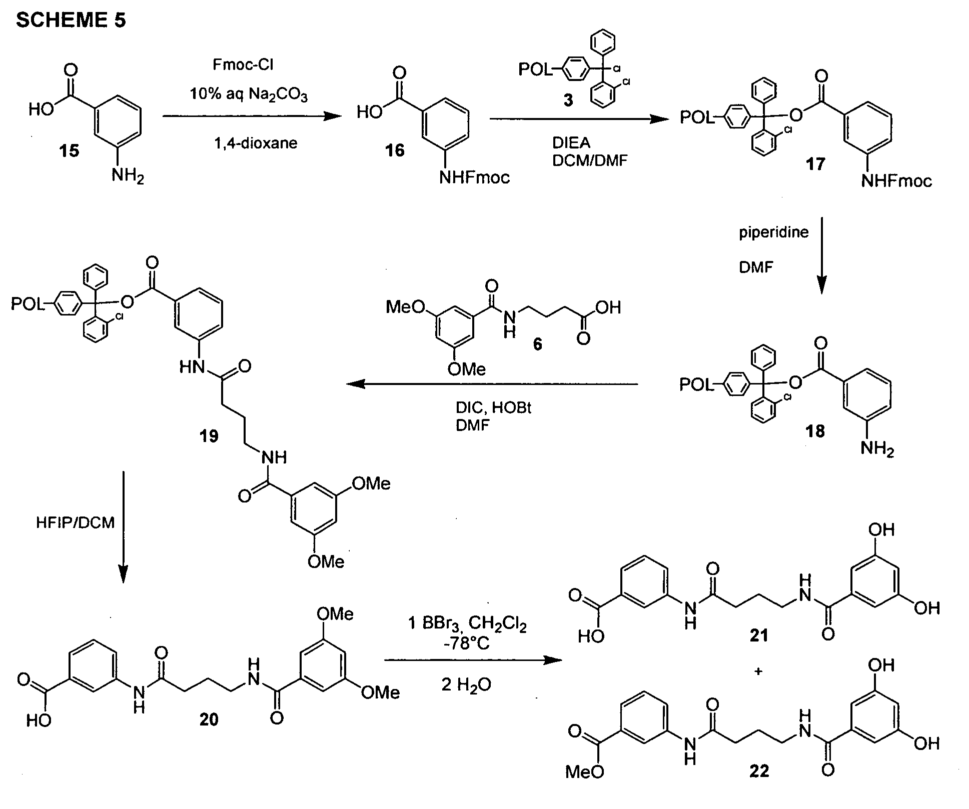

**Step 1:** Dissolve 3-Aminobenzoic acid ((15); 1.00g, 7.30mmol) in 1,4-dioxane (12.0mL) and 10% aqu. Na₂CO₃ (20.8mL) and add Fmoc-Cl (2.26g, 8.76mmol). Stir the reaction mixture for 2.5h at rt. Add 1M aqu HCl (42.0mL) to the mixture and extract with EtOAc (3 times). Wash the combined organic layers with 1M aqu HCl, water and brine, extract the combined aqu layers once again with EtOAc, dry the combined organic layers with Na₂SO₄ and remove solvent under reduced pressure. The left crude product is washed with ice cold EtOAc and dried in oil pump vacuum to obtain (16) as a white solid (1.61g, 61 %). No further purification. [M. Nichifor; E. H. Schacht; Tetrahedron; 1994; 50; 12; 3747-3760]. ¹H NMR (400MHz, DMSO-d₆): 4.31 (t, 1 H, J= 6.6Hz); 4.49 (d, 2 H, *J* = 6.6Hz); 7.31-7.45 (m, 5 H); 7.56 (d, 1 H, *J* = 7.6Hz); 7.60-7.70 (br.m, 1 H); 7.75 (d, 2 H, *J* = 7.3Hz); 7.90 (d, 2 H, *J* = 7.3Hz); 8.11 (s, 1 H); 9.88 (s, 1 H).
**Step 2:** Dissolve (16) (180mg, 0.5mmol) in DCM (0.5mL) and DMF (0.25mL) at rt in a pre-dried tube, add DIEA (0.52mL, 1.5mmol) and add this solution to 2-chlorotritylchlorid-polystyrene (3) (82mg, 0.13mmol (loading 1.6mmol/g)) which is preswollen before in DCM (0.15mL). Shake the reaction suspensions for 14h at rt. The resin bound product (17) is washed with DCM/MeOH/DIEA (17+2+1, 3 times), DCM (once), DMF (3 times) and again DCM (twice) and dried in vacuum. [i.e. Novabiochem® 2000 Catalog; 2000; S15-S18].
**Step 3:** Suspend complete amount of resin (17) from step 2 in DMF (0.4mL) and piperidine (0.1mL) and shake it for 1.5h at rt. Washed resin bound product (18) with DMF (3 times) and DCM (3 times for 30min) and dry in vacuum.
**Step 4:** Complete amount of resin (18) from step 3 is preswollen in DMF (0.8mL) for 20min. Dissolve acid (6) (158mg, 0.59mmol) and HOBt (90mg, 0.59mmol) in DMF (2.8mL) at rt, add DIC (75mg, 0.59mmol), stir for 15min and add this solution to the preswollen resin (18). Shake the resin suspension gently for 20h at rt. Wash resin bound product (19) with DMF (3 times) and DCM (4 times) and dry in vacuum.
**Step 5:** Suspend complete amount of resin (19) from step 4 in 1.5mL of DCM+HFIP (2+1) and shake for 45min at rt. Filter the remaining solution of and wash resin once with DCM. Repeat cleavage procedure and washing once. Evaporate solvent of the combined filtrates under reduced pressure to afford crude product (20). No further purification.
**Step 6:** (The following reaction is done in an anhydrous N₂ atmosphere.) Suspend complete amount of crude (20) from step 5 in anhydrous DCM (2.0mL), cooled it to -40°C and added BBr₃ (0.15mL, 1.59mmol). Shake the reaction suspension for 1h at -40°C, 2h at -25°C and 30min at +5°C. Add dropwise water under vigorous stirring followed by MeOH. Remove solvent under reduced pressure. Purify the crude product by preparative RP HPLC (gradient, water/MeCN 95:5) to obtain 3-[4-(3,5-Dihydroxy-benzoylamino)-butyrylamino]-benzoic acid (21) (8.9mg, 19% over 5 steps) and 3-[4-(3,5-Dihydroxy-benzoylamino)-butyrylamino]-benzoic acid methyl ester (22) (3.0mg, 6% over 5 steps) as white solids both. ¹H NMR (400MHz, CD₃OD) (21): 2.03 (quint, 2 H, J= 7.1 Hz); 2.51 (t, 2H, *J* = 7.5Hz); 3.47 (t, 2 H, *J* = 6.7Hz); 6.44 (br.s, 1 H); 6.75 (br.s, 2 H); 7.44 (dd, 1 H, *J₁* = 8.3Hz, *J₂* = 7.8Hz); 7.78 (d, 1 H, *J* = 7.8Hz); 7.85 (d, 1 H, *J* = 8.6Hz); 8.26 (br.s, 1 H); (22): 2.03 (quint, 2 H, *J* = 6.9 Hz); 2.51 (t, 2H, *J* = 7.5Hz); 3.47 (t, 2 H, J= 6.9Hz); 3.94 (s, 3 H); 6.43 (br.t, 1 H, *J* = 2.0Hz); 6.74 (d, 2 H, *J* = 2.0Hz); 7.44 (t, 1 H, *J* = 8.1Hz); 7.77 (d, 1 H,, *J* = 7.6Hz); 7.83 (br.d, 1 H, *J* = 8.1Hz); 8.28 (br.s, 1 H).

### EXAMPLE 2

### {4-[4-(3,5-Dihydroxy-benzoylamino)-butyryl]-piperazin-1-yl}-acetic acid (23)

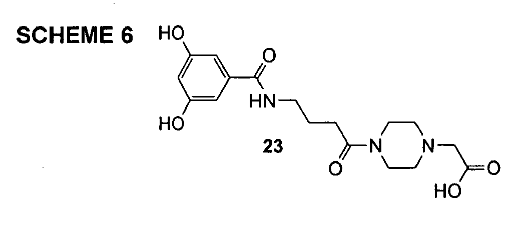

According to the procedure described in EXAMPLE 1 {4-[4-(3,5-Dihydroxy-benzoylamino)-butyryl]-piperazin-1-yl}-acetic acid (23) is obtained as a yellowish oil (6.8mg, 14% over 5 steps). ¹H NMR (400MHz, CD₃OD): 1.96 (quint, 2 H, *J* = 6.7 Hz); 2.54 (t, 2H, *J* = 6.9Hz); 3.41-3.51 (m, 4 H); 3.90 (br.s, 4 H); 4.12 (s, 2 H); 6.46 (br.s, 1 H); 6.74 (d; 2 H, *J* = 2.0Hz).

### EXAMPLE 3

### 4-[4-(3,5-Dihydroxy-benzoylamino)-butyrylamino]-benzoic acid (24)

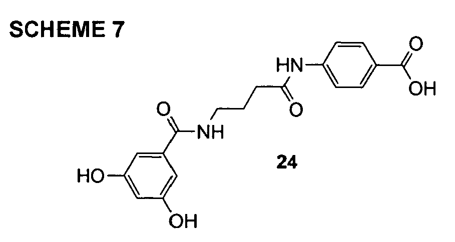

According to the procedure described in EXAMPLE 1 4-[4-(3,5-Dihydroxy-benzoylamino)-butyrylamino]-benzoic acid (24) is obtained as a white solid (1.2mg, 2% over 5 steps). ¹H NMR (400MHz, CD₃OD): 2.03 (quint, 2 H, J= 6.9Hz); 2.52 (t, 2H, *J* = 7.8Hz); 3.47 (t, 2H, *J* = 6.6Hz); 6.44 (br.s, 1 H); 6.75 (br.s, 2 H); 7.71 (d, 2 H, *J* = 8.3Hz); 7.99 (d, 2 H, *J* = 8.3 Hz).

### EXAMPLE 4

### 5-{4-[2-(2,4-Dimethoxy-phenyl)-acetylamino]-phenyl}-2-methyl-furan-3-carboxylic acid ethyl ester (27) and 5-{4-[2-(2,4-Dimethoxy-phenyl)-acetylamino]-phenyl}-2-methyl-furan-3-carboxylic acid (28)

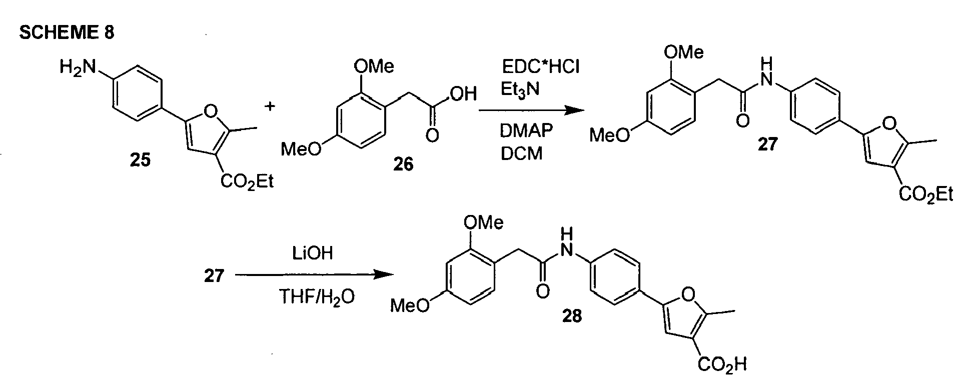

**Step 1:** (The following reaction is done in an anhydrous N₂ atmosphere.) Dissolve EDC hydrochloride (117mg, 0.61mmol) and triethylamine (85µL, 0.61mmol) in anhydrous dichloromethane (2.0mL) and stir for 5min at rt. Add acid (26) (84mg, 0.43mmol) and DMAP (8mg, 0.06mmol) and stir for 10min. Add ethyl ester (25) (100mg, 0.41mmol) and stir the reaction solution overnight at rt. Hydrolize the reaction solution with saturated aqu. NH₄Cl followed by water, separate layers, extract aqu. layer with dichloromethane (3 times) and wash the combined organic layers with water and brine and dry with Na₂SO₄. Remove solvent under reduced pressure. Purify crude product by preparative radial chromatography (silica gel 60PF, EtOAc/CyH 1+1) to obtain 5-{4-[2-(2,4-Dimethoxyphenyl)-acetylamino]-phenyl}-2-methyl-furan-3-carboxylic acid ethyl ester (27) as a yellow solid (153mg, 88%). [K. C. Nicolaou; P. S. Baran; Y.-L. Zhong; K. Sugita; J. Am. Chem. Soc.; 2002; 124; 10; 2212-2220]. ¹H NMR (400MHz, CDCl₃): 1.34 (t, 3 H, *J* = 7.2Hz); 2.61 (s, 3 H); 3.63 (s, 2 H); 3.81 (s, 3 H); 3.89 (s, 3 H); 4.28 (q, 2 H, *J*= 7.2Hz); 6.48-6.53 (m, 2 H); 6.77 (s, 1 H); 7.19 (d, 1 H, *J* = 8.1Hz); 7.42 (d, 2 H, *J* = 8.6Hz); 7.52 (d, 2 H, *J* = 8.8Hz); 7.60 (br.s, 1 H).
**Step 2:** Dissolve 5-{4-[2-(2,4-Dimethoxy-phenyl)-acetylamino]-phenyl}-2-methylfuran-3-carboxylic acid ethyl ester (27) (150mg, 0.35mmol) in MeOH (0.5mL) and THF (8mL) at rt and add 1M aqu LiOH (3.6mL, 3.6mmol). Stir reaction mixture 18h at rt. Quench reaction mixture (cooling bath) with 2M aqu. HCl. Extract the mixture with EtOAc (3x), wash the combined organic layer with brine and dry with Na₂SO₄ to obtain 5-{4-[2-(2,4-Dimethoxy-phenyl)-acetylamino]-phenyl}-2-methyl-furan-3-carboxylic acid (28) (136mg, 97%) as a white solid. ¹H NMR (400MHz, CD₃OD): 2.66 (s, 3 H); 3.65 (s, 2 H); 3.83 (s, 3 H); 3.86 (s, 3 H); 6.54 (dd, 1 H, *J₁* = 8.3Hz, *J₂* = 2.3Hz); 6.59 (d, 1 H, *J* = 2.3Hz); 6.91 (d, 1 H); 7.18 (d, 1 H, *J* = 8.3Hz); 7.64 (s, 4 H).

### EXAMPLE 5

### 5-{4-[2-(2,4-Dihydroxy-phenyl)-acetylamino]-phenyl}-2-methyl-furan-3-carboxylic acid (29)

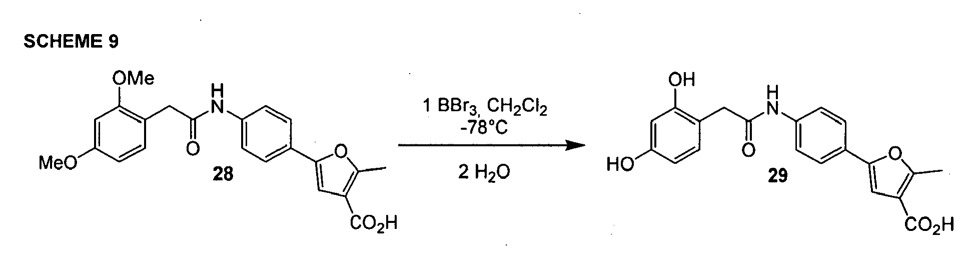

(The following reaction is done in an anhydrous N₂ atmosphere.) Dissolve 5-{4-[2-(2,4-Dimethoxy-phenyl)-acetylamino]-phenyl}-2-methyl-furan-3-carboxylic acid (28) (100mg, 0.25mmol) in anhydrous DCM (2.5mL), cool the solution to -78°C and add dropwise BBr₃ (95µL, 1.01mmol). Stir the reaction mixture for 30min at -78°C and after slowly warming up for additional 2h at rt. Add dropwise ice water, separate layers and extract aqu. layer with DCM (3 times). Wash combined organic layer with brine and dry with Na₂SO₄.. Purify the crude product by preparative RP HPLC (gradient, water/CH₃CN 95:5 to 5:95) to obtain 5-{4-[2-(2,4-Dihydroxy-phenyl)-acetylamino]-phenyl}-2-methyl-furan-3-carboxylic acid (29) (30mg, 32%). ¹H NMR (400MHz, CD₃OD): 2.65 (s, 3 H); 3.62 (s, 2 H); 6.33 (dd, 1 H, *J₁* = 8.3Hz, *J₂* = 2.3Hz); 6.39 (d, 1 H, *J* = 2.3Hz); 6.89 (s, 1 H); 7.02 (d, 1 H, *J* = 8.3Hz); 7.62 (s, 4 H).

### EXAMPLE 6

### 5-{4-[2-(3,5-Dimethoxy-phenyl)-acetylamino]-phenyl}-2-methyl-furan-3-carboxylic acid (30)

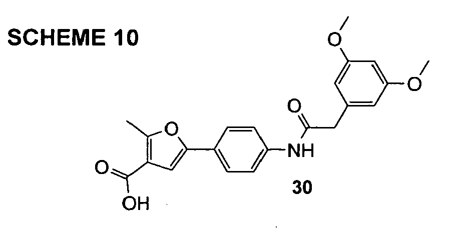

According to the procedure described in EXAMPLE 4 5-{4-[2-(3,5-Dimethoxy-phenyl)-acetylamino]-phenyl}-2-methyl-furan-3-carboxylic acid (30) is obtained as a white solid (138mg, 85% over 2 steps). ¹H NMR (400MHz, CD₃OD): 2.66 (s, 3 H); 3.65 (s, 2 H); 3.81 (s, 6 H); 6.43 (t, 1 H, *J* = 2.0Hz); 6.58 (d, 2 H, *J* = 2.0Hz); 6.92 (s, 1 H); 7.65 (s, 4 H).

### EXAMPLE 7

### 5-{4-[2-(3,5-Dihydroxy-phenyl)-acetylamino]-phenyl}-2-methyl-furan-3-carboxylic acid (31)

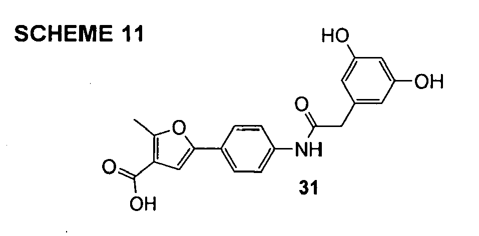

According to the procedure described in EXAMPLE 5 5-{4-[2-(3,5-Dihydroxy-phenyl)-acetylamino]-phenyl}-2-methyl-furan-3-carboxylic acid (31) is obtained as a white solid (57mg, 55% yield). ¹H NMR (400MHz, CD₃OD): 2.66 (s, 3 H); 3.57 (s, 2 H); 6.22 (t, 1 H, *J* = 2.0Hz); 6.35 (d, 2 H, *J* = 2.0Hz); 6.91 (s, 1 H); 7.65 (s, 4 H).

The compounds referred to in the following SCHEME 12 are those compounds referred to as the particularly preferred compounds herein.

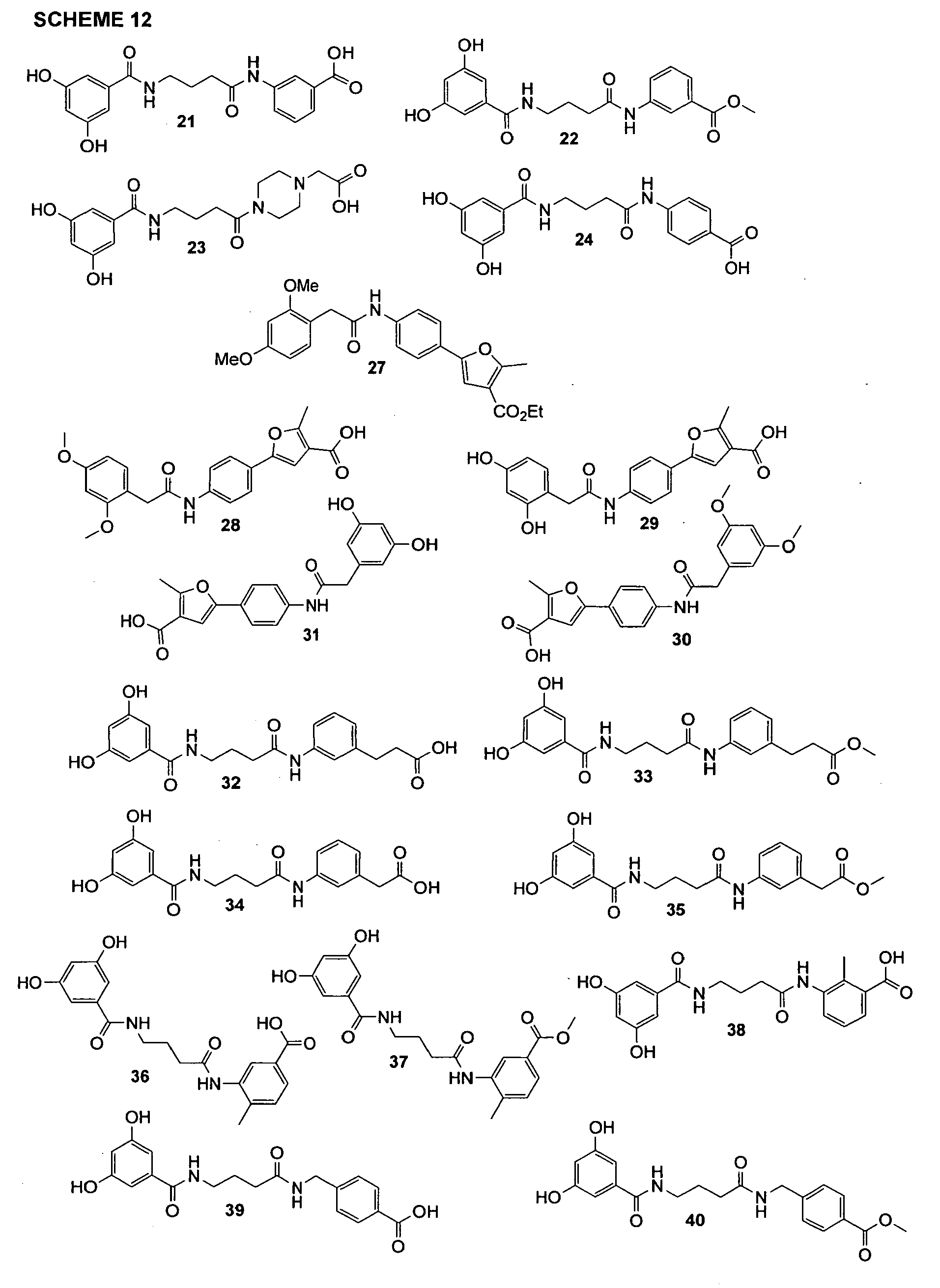

### Sialyl Lewis^{x} Tyrosine Sulfate Assay (sLe^{x} TSA):

Compounds of the present invention are assayed on a molecular level for their ability to inhibit the binding of P-, L-, or E-selectin chimeric molecules to sLe^{x} and tyrosinesulfate residues linked to a polymeric matrix as a PSGL-1 substitute. Selected IC₅₀-values are determined.
Microtiter plates are coated overnight in carbonate buffer pH9,6 with goat anti human Fc mAB (10 µg/ml). After washing in assay buffer (25mM 4-(2-hydroxyethyl)-1-piperazineethanesulfonic acid (HEPES), 150mM NaCl, 1mM CaCl₂ pH7,4) and blocking (3% bovine serum albumin (BSA) in assay buffer) plates are incubated for 2h at 37°C with human P-Selectin-IgG-chimera (0,61nM respectively 150ng/mL) or human L-Selectin-IgG-chimera (0,61nM respectively 89ng/mL) or human E-Selectin-IgG-chimera (0,61nM respectively 131 ng/mL). 5µl of sLe^{x} -tyrosine sulfate polyacrylamide (1mg/ml) carrying 15% sLe^{x}, 10% Tyrosine-sulfate and 5% biotin is complexed with 20µl Streptavidin-Peroxidase solution (1mg/ml) and 25µl assay buffer without CaCl₂. For use in the assay, the ligand complex is diluted 1:10000 in assay buffer and further diluted 1:1 with varying amounts of compounds in assay buffer incl. 2%DMSO. This mixture is added to the wells precoated with E- or P-selectin. After incubation for 2h at 37°C, wells are washed for six times with in assay buffer incl. 0,005% Polyoxyethylenesorbitan monolaurate (TWEEN 20), developed for 10-15min with 20µl 3,3',5,5'-tetramethylbenzidine (TMB)/H₂O₂ substrate solution and stopped with 20µl 1M H₂SO₄. Bound sLe^{x} -Tyrosine sulfate ligand complex is determined by measuring optical density at 450nm vs. 620nm in a Fusion alpha-FP reader (sold from Packard Bioscience, Dreieich, Germany).

**Results from sLe^{x}TSA: in vitro inhibition Data for E-/ P-/ L-Selectin at 100µM**

| **Compound** | **E-Selectin [% inhib.]** | **P-Selectin [% inhib.]** | **L-Selectin [% inhib.]** |
|---|---|---|---|
| **Bimosiamose** | 3.9 | 22.6 | 6.2 |
| **21** | 40.7 | 3.0 | 18.7 |
| **22** | 42.7 | 22.7 | 21.3 |
| **23** | 10.5 | 18.9 | 11.6 |
| **24** | 35.9 | 0.5 | 5.9 |
| **32** | 35.0 | 1.4 | 13.1 |
| **33** | 38.6 | 10.6 | 27.3 |
| **34** | 41.0 | 12.9 | 18.1 |
| **35** | 40.8 | 15.6 | 19.4 |
| **36** | 35.8 | 1.2 | 6.8 |
| **37** | 38.9 | 5.2 | 9.5 |
| **38** | 40.9 | 9.4 | 19.3 |
| **39** | 24.8 | 2.9 | 10.9 |
| **40** | 34.4 | 28.2 | 31.1 |

**Results from sLe^{x}TSA: IC₅₀ Data for E-/ P-/ L-Selectin**

| **Compound** | **IC₅₀ E-Selectin [µM]** | **IC₅₀ P-Selectin [µM]** | **IC₅₀ L-Selectin [µM]** |
|---|---|---|---|
| **Bimosiamose** | >500 | 95.0 | >500 |
| **28** | 200.7 | 237.7 | 318.6 |
| **30** | >500 | 133.5 | 376.1 |

### Flow Chamber Assay / Cell Adhesion and Rolling under Flow Conditions

To assess the capability of compounds to inhibit cell binding under dynamic conditions resembling the flow in a blood vessel, flow chamber assays addressing/ testing binding of HL-60 cells / various cell lines to P-selectin, L-selectin and E-selectin chimeric molecules are performed.
Cell attachment under flow conditions are determined using a parallel flow chamber system. A 35mm polystyrene culture dish is coated for 1 hour at room temperature with coating buffer (50mM tris-(hydroxymethyl) aminomethane buffer (Tris), 150 mM NaCl, 2 mM CaCl₂; pH 7,4) containing human E- or P-selectin-IgG chimera at concentrations of 2,5µg/ml or 10µg/ml, respectively. After removal of the coating solution non specific binding sites are blocked for an additional hour with 1% BSA in coating buffer at room temperature. After washing with assay buffer ("Roswell Park Memorial Institute 1640" (RPMI 1640) + 10mM HEPES) the dish is fitted into a parallel plate laminar flow chamber (sold from Glycotech, Rockville, MD) and mounted on an inverted phase-contrast microscope (sold from Olympus, Hamburg, Germany) equipped with a CCD camera (JVC) that is connected to a PC. Employing a peristaltic pump (sold from Ismatec, Wertheim-Mondfeld, Germany) the re-circulating system is equilibrated with assay buffer containing 125µM compound or vehicle control (DMSO). Cells (1 million / ml) are added to the chamber and allowed to distribute for 2 minutes at a high flow rate. The flow rate is then decreased resulting in a calculated flow shear of 1 dyne/cm². Video sequences of 10 low power fields are digitally recorded after 5 minutes continuous flow. The percentage of inhibition is calculated from the mean number of cells per field that attached to the coated dish surface in the presence versus absence of compound of at independent experiments.

### Data from Flow Chamber Assay for E- and P-Selectin

Values are given as normalized ratios of %-inhibition of compound x divided by %-inhibition of bimosimaose.

| **Compound** | **E-Selectin [Ratio]** | **P-Selectin [Ratio]** |
|---|---|---|
| **28** | 0.99 | n.s. |
| **29** | 1.31 | 0.74 |
| **30** | 1.03 | n.s. |
| **31** | 1.16 | 0.60 |

## Claims

1. Pharmaceutical compositions comprising at least one compound of the formulas (Ia) or (Ib) or (IIa) or (IIb) and a pharmaceutically acceptable carrier which is useful in a medicine, 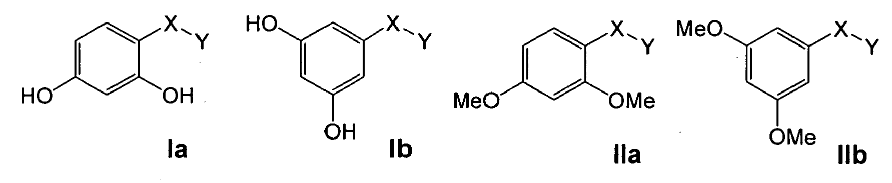 wherein the symbols and substituents have the following meaning
-X- = 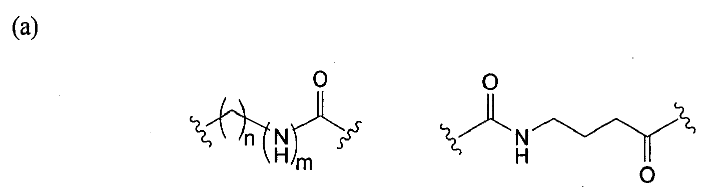 with m = 0,1; n = an integer from 1 to 3 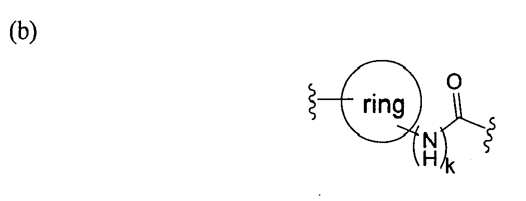 wherein "ring" is 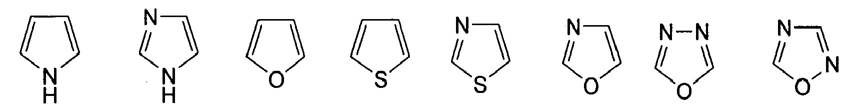 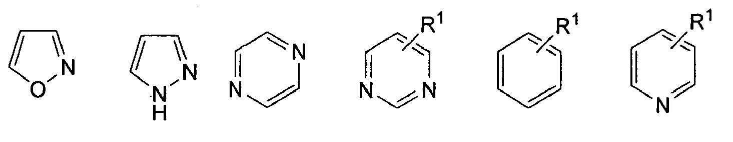 and with R¹ being H, NO₂, CF₃, F, Cl, Br, I, CN, CH₃, NH₂, NHAlkyl, NHAryl, NHAcyl and k = 0,1 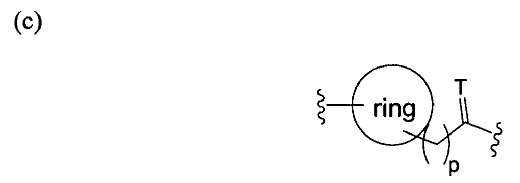 T being O, S or [H,H]; p = 0,1,2, 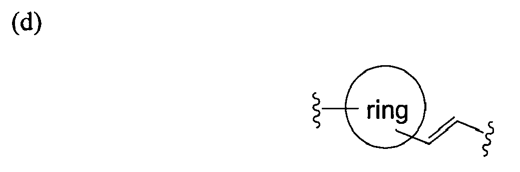 the double bond is either *E*- or *Z*-configurated 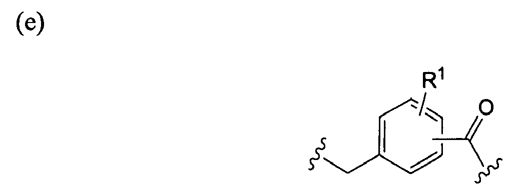 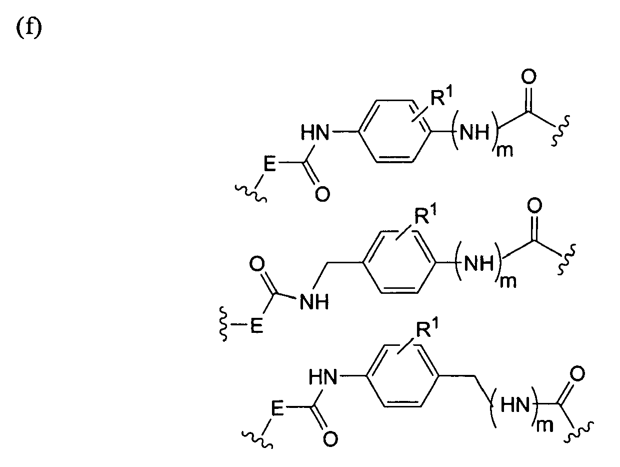 with -E- being -(CH₂-)_{q}NH- and q = 0, 1, 2, 3
-Y= 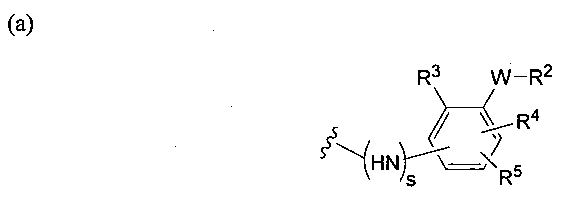 with
s being 0 or 1,
R² being CO₂H, CO₂Alkyl, CO₂Aryl, CO₂NH₂, CO₂Aralkyl, SO₃H, SO₂NH₂, PO(OH)₂, 1-H-tetrazolyl-, CHO, COCH₃, CH₂OH, NH₂, NHAlkyl, N(Alkyl)Alkyl', OCH₃, CH₂OCH₃, SH, F, Cl, Br, I, CH₃, CH₂CH₃, CN, CF₃
R³ independently from R² being H, CH₃, CH₂CH₃, CF₃, F, Cl, Br, I, CN, NO₂ and
R⁴ independently from R² and R³ being H, CH₃, CH₂CH₃, CF₃, F, Cl, Br, I, CN, NO₂, R²
R⁵ being H, NO₂, CF₃, F, Cl. Br, I, CN, CH₃, OCH₃, SH, NH₂
and -W- = -(CH₂-)ᵥ, cis-CH=CH- or *trans*-CH=CH-, and v being 0,1,2;
in case that -W- is cis-CH=CH- or *trans*-CH=CH-, R² must not be NH₂ or SH; 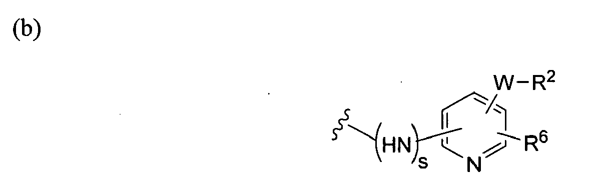
R⁶ independently from R² being H, F, Cl, Me, tert-Bu, CN, NH₂ 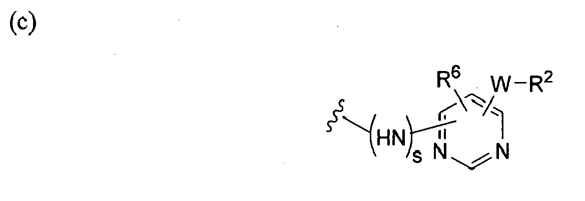 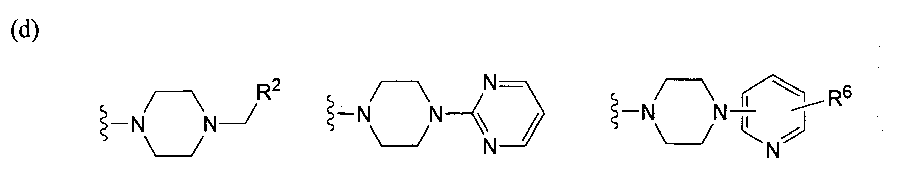 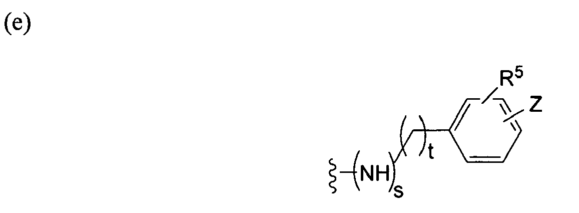 with t being 0,1,2 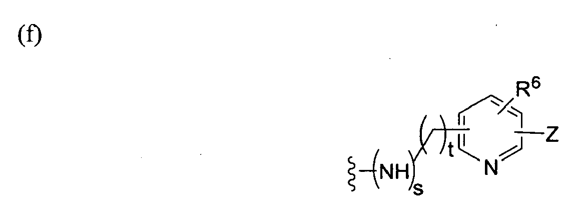 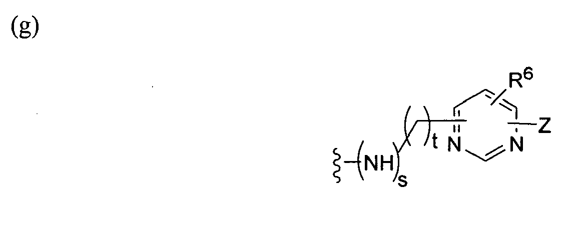
-Z= 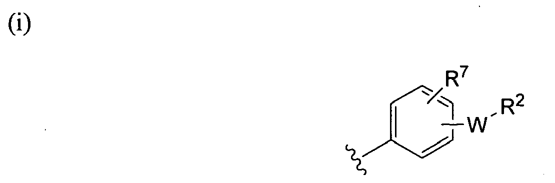 R⁷ independently from R² being H, NO₂, CF₃, F, Cl. Br, I, CN, CH₃, OCH₃, SH, NH₂, 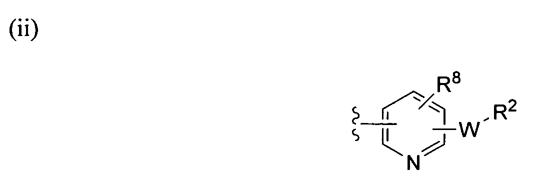 R⁸ independently from R² being H, F, Cl, Me, tert-Bu, CN, NH₂ 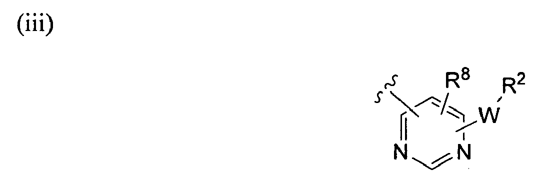 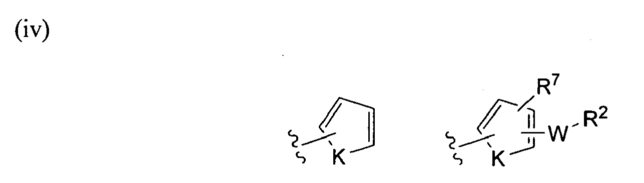 with K = NH, NMe, O, S 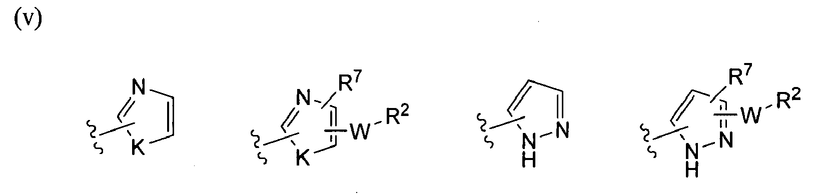 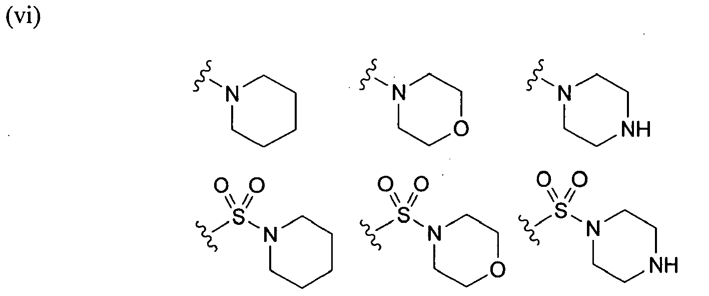
(vii) -W-R²
or the pharmaceutically acceptable salts, esters or amides and prodrugs of the above identified compounds of formulas (Ia) or (Ib) or (IIa) or (IIb).

2. Pharmaceutical compositions according to claim 1, wherein the compounds are defined by formulas (IIIa) or (IIIb) or (IVa) or (IVb) 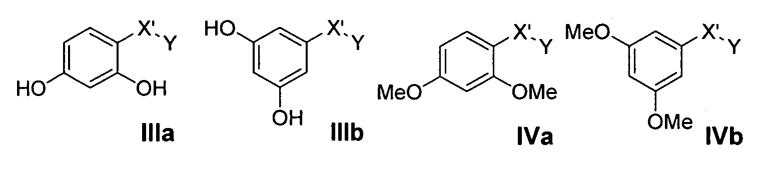 wherein -Y and -X'- is X (a), X (b), X (c), and X (d) is as defined in claim 1.

3. Pharmaceutical compositions according to claim 2, wherein the compounds are defined by formulas (A 1) or (A2) or (B 1) or (B2) or (C 1) or (C2) or (D 1) or (D2) 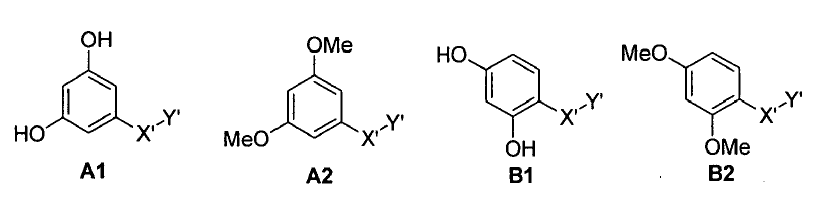 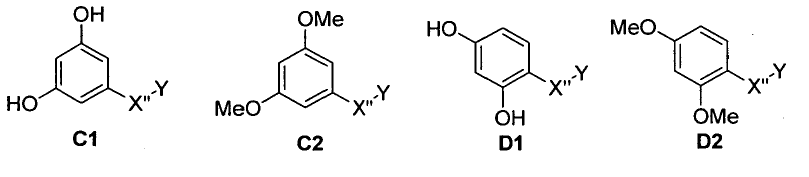 wherein -X'- and -Y are as defined in claim 2 and wherein -X"- is 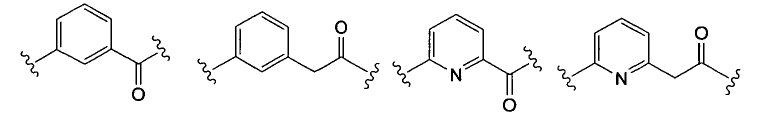 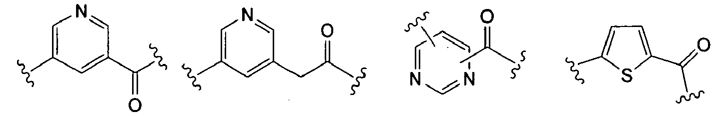 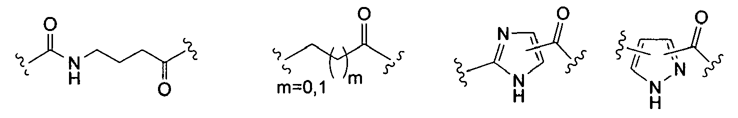 and wherein -Y' is 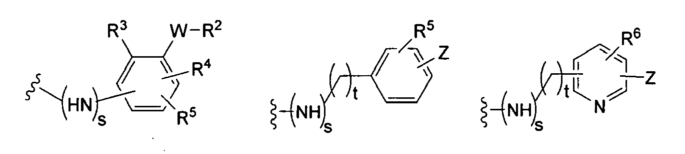 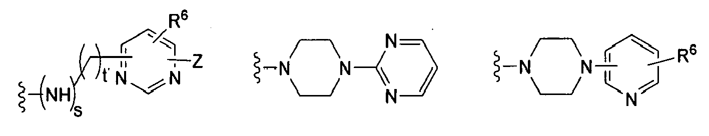 wherein all indices, symbols and substituents are as defined in claim 1.

4. Pharmaceutical compositions according to claim 3, wherein the compounds are defined by formulas (E1) or (E2) or (F1) or (F2). 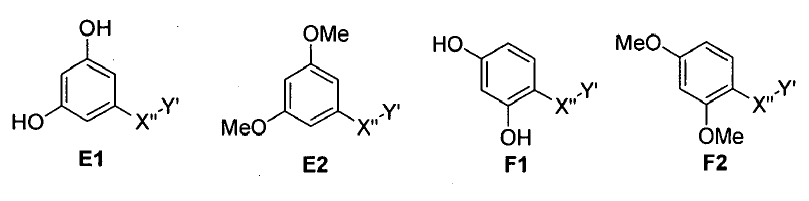 wherein -X"- and -Y' are as defined in claim 3.

5. Pharmaceutical compositions according to claim 1, wherein the compounds are defined by formulas (G1) or (G2) or (H1) or (H2) 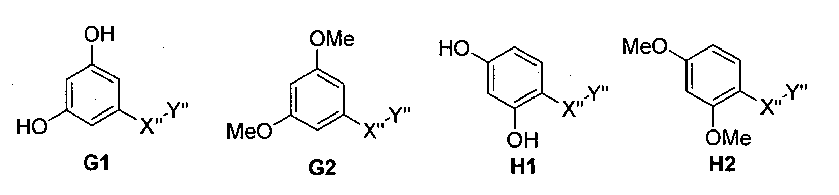 wherein -X"- is as defined in claim 3 and -Y" is 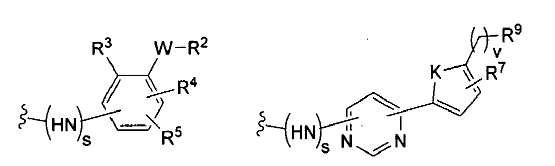 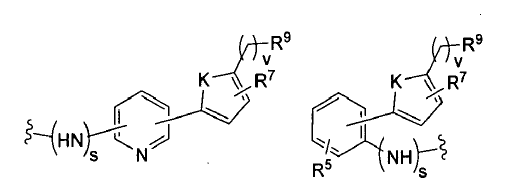 with R⁹ being CO₂H, CO₂alkyl, CO₂aryl, CO₂NH₂, CO₂aralkyl, CH₂SO₃H, CH₂SO₂NH₂, CH₂PO(OH)₂, 1-H-tetrazolyl, CHO, COCH₃, CH₂OH, CH₂NH₂, CH₂NHalkyl, CH₂N(alkyl)alkyl', CH₂OCH₃, CH₂SH,
wherein all indices, symbols and substituents are as defined in claim 1.

6. Chemical compounds having the general structure of formula (E1), (E2), (F1), (F2), (G1), (G2), (H1) or (H2) according to claim 4 or 5.

7. Method of modulating the binding of P-selectin, L-selectin or E-selectin to PSGL-1 or PSGL-1-like ligands or any ligands bearing sLe^{x} or sLe^{a} and tyrosinesulfate motifs comprising the step of administering to a patient an effective amount of at least one compound having the structure of formulas (Ia) or (Ib) or (IIa) or (IIb) as defined in claim 1.

8. Use of compounds having the structure of formulas (Ia) or (Ib) or (IIa) or (IIb) as defined in claim 1 for the preparation of a medicine for the treatment of a patient, modulating the binding of P-selectin, L-selectin or E-selectin to PSGL-1 or PSGL-1-like ligands or any ligands bearing sLe^{x} or sLe^{a} and tyrosinesulfate motifs.

9. Use of compounds having the structure of formulas (Ia) or (Ib) or (IIa) or (IIb) as defined in claim 1 for the preparation of a medicine for the treatment, diagnosis or prophylaxis of inflammatory disorders and other medical conditions where selectin mediated processes play a role.

10. Use of compounds having the structure of formulas (Ia) or (Ib) or (IIa) or (IIb) as defined in claim 1 for the preparation of a vehicle for drug targeting of diagnostics or therapeutics.or therapeutics.

## Amended claims

### Amended claims in accordance with Rule 86(2) EPC.

**1.** Pharmaceutical compositions comprising at least one compound of the formulas (Ia) or (Ib) or (IIa) or (IIb) and a pharmaceutically acceptable carrier which is useful in a medicine, 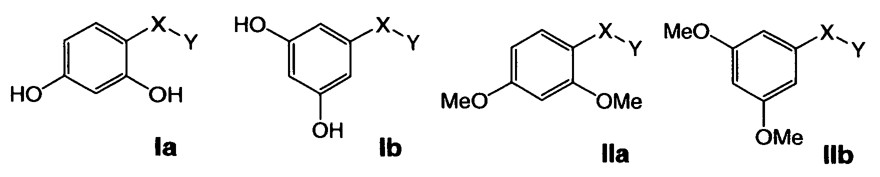 wherein the symbols and substituents have the following meaning
-X- = 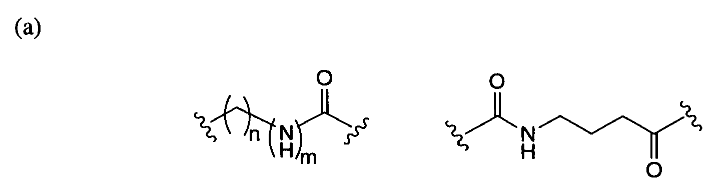 with m = 0,1; n = an integer from 1 to 3 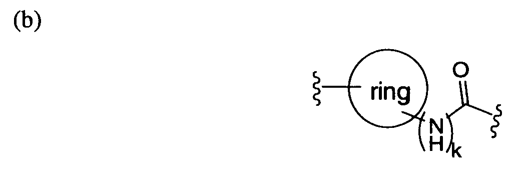 wherein "ring" is 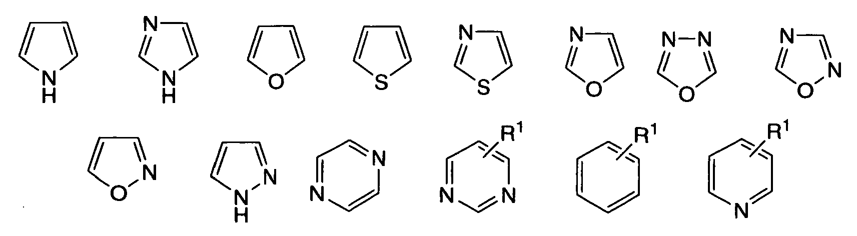 and with R¹ being H, NO₂, CF₃, F, Cl, Br, I, CN, CH₃, NH₂, NHAlkyl, NHAryl, NHAcyl and k = 0,1 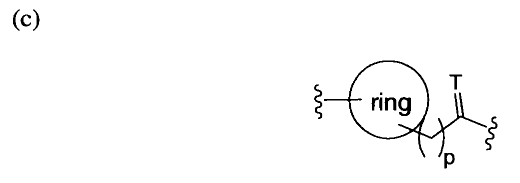 T being O, S or [H,H]; p = 0,1,2, 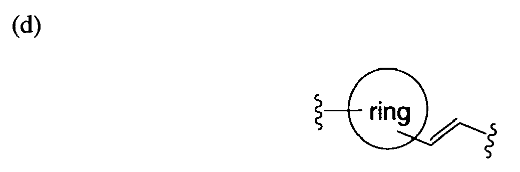 the double bond is either E- or Z-configurated
-Y = 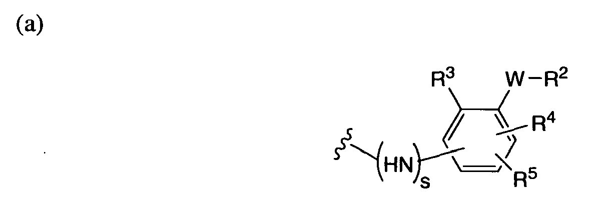 with s being 0 or 1,
R² being CO₂H, CO₂Alkyl, CO₂Aryl, CO₂NH₂, CO₂Aralkyl, SO₃H, SO₂NH₂, PO(OH)₂, 1-H-tetrazolyl-, CHO, COCH₃, CH₂OH, NH₂, NHAlkyl, N(Alkyl)Alkyl', OCH₃, CH₂OCH₃, SH, F, Cl, Br, I, CH₃, CH₂CH₃, CN, CF₃
R³ independently from R² being H, CH₃, CH₂CH₃, CF₃, F, Cl, Br, I, CN, NO₂ and
R⁴ independently from R² and R³ being H, CH₃, CH₂CH₃, CF₃, F, Cl, Br, I, CN, NO₂, R₂
R⁵ being H, NO₂, CF₃, F, Cl. Br, I, CN, CH₃, OCH₃, SH, NH₂ and -W- = -(CH₂-)ᵥ, cis-CH=CH- or *trans*-CH=CH-*,* and v being 0,1,2;
in case that -W- is cis-CH=CH- or *trans-*CH=CH-*,* R² must not be NH₂ or SH; 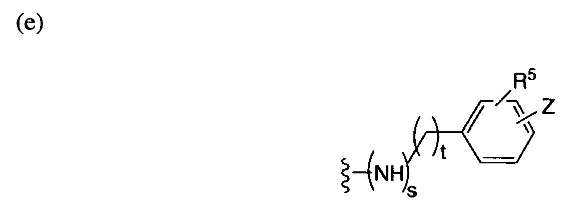 with t being 0,1,2 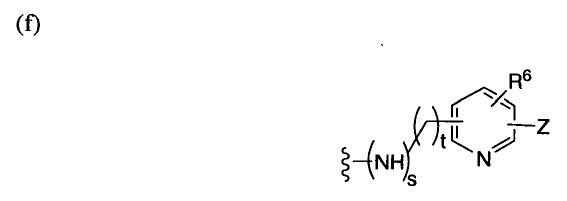 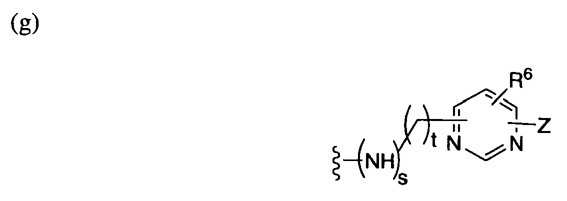 -Z = 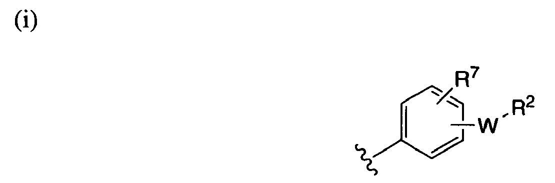 R⁷ independently from R² being H, NO₂, CF₃, F, Cl. Br, I, CN, CH₃, OCH₃, SH, NH₂, 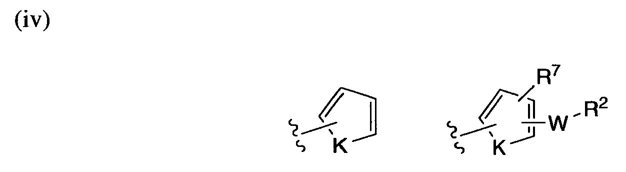 with K = NH, NMe, O, S 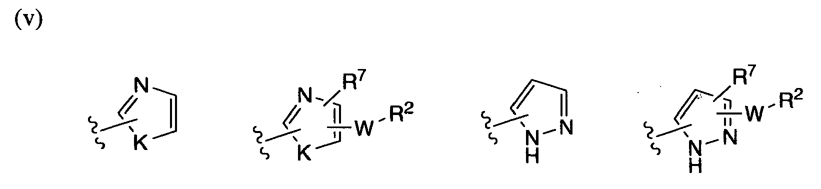 or the pharmaceutically acceptable salts, esters or amides and prodrugs of the above identified compounds of formulas (Ia) or (Ib) or (IIa) or (IIb).

**2.** Pharmaceutical compositions according to claim 1, wherein the compounds are defined by formulas (IIIa) or (IIIb) or (IVa) or (IVb) 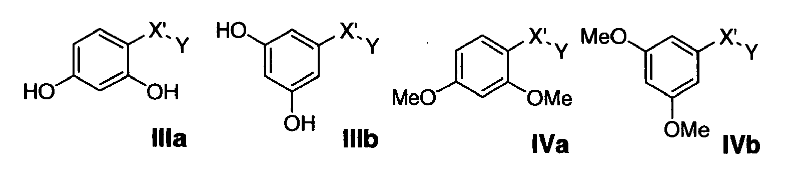 wherein -Y is defined as in claim 1 and -X'- is X (a), X (b) and X (c) as defined in claim 1.

**3.** Pharmaceutical compositions according to claim 2, wherein the compounds are defined by formulas (A1) or (A2) or (B1) or (B2) or (C1) or (C2) or (D1) or (D2) 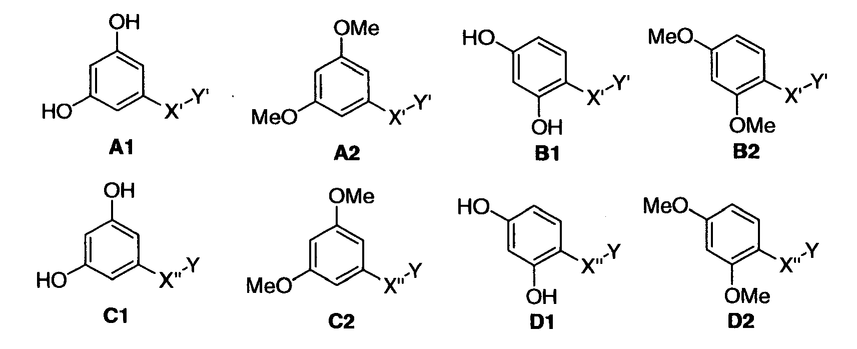 wherein -X'- and -Y are as defined in claim 2 and wherein -X"- is 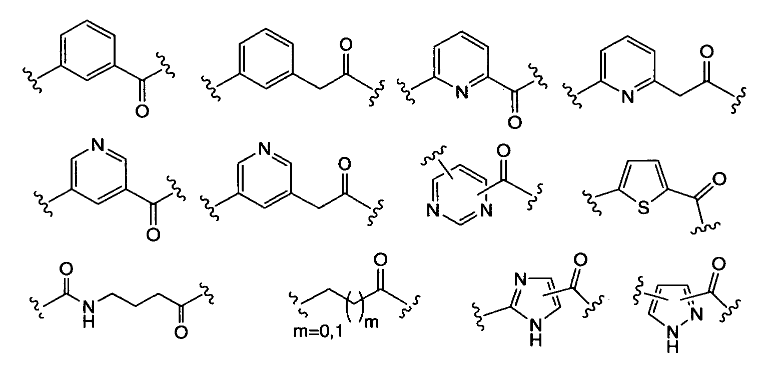 and wherein -Y' is 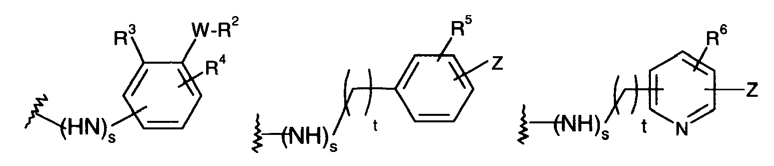 wherein all indices, symbols and substituents are as defined in claim 1.

**4.** Pharmaceutical compositions according to claim 3, wherein the compounds are defined by formulas (E1) or (E2) or (F1) or (F2). 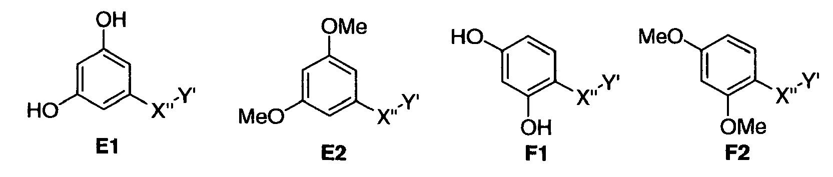 wherein -X"- and -Y' are as defined in claim 3.

**5.** Pharmaceutical compositions according to claim 1, wherein the compounds are defined by formulas (G1) or (G2) or (H1) or (H2) 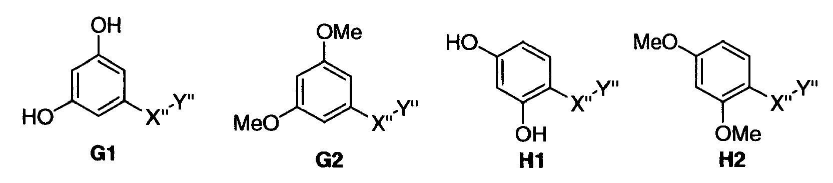 wherein -X"- is as defined in claim 3 and -Y" is 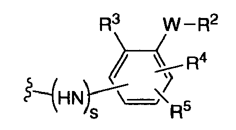 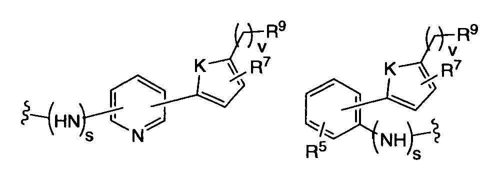 with R⁹ being CO₂H, CO₂alkyl, CO₂aryl, CO₂NH₂, CO₂aralkyl, CH₂SO₃H, CH₂SO₂NH₂, CH₂PO(OH)₂, 1-H-tetrazolyl, CHO, COCH₃, CH₂OH, CH₂NH₂, CH₂NHalkyl, CH₂N(alkyl)alkyl', CH₂OCH₃, CH₂SH,
wherein all indices, symbols and substituents are as defined in claim 1.

**6.** Chemical compounds having the general structure of formula (E1), (E2), (F1), (F2), (G1), (G2), (H1) or (H2) according to claim 4 or 5.

**7.** Use of compounds having the structure of formulas (Ia) or (Ib) or (IIa) or (IIb) as defined in claim 1 for the preparation of a medicine for the treatment of Chronic Obstructive Pulmonary Disease (COPD), acute lung injury (ALI), cardiopulmonary bypass, acute respiratory distress syndrome (ARDS), Crohn's disease, septic shock, sepsis, chronic inflammatory diseases such as psoriasis, atopic dermatitis, and rheumatoid arthritis, and reperfusion injury that occurs following heart attacks, strokes, atherosclerosis, and organ transplants, traumatic shock, multi-organ failure, autoimmune diseases like multiple sclerosis, percutaneous transluminal angioplasty, asthma and inflammatory bowel disease.

**8.** Use of compounds having the structure of formulas (Ia) or (Ib) or (IIa) or (IIb) as defined in claim 1 for the preparation of a medicine for the treatment, diagnosis or prophylaxis of inflammatory disorders.

**9.** Use of compounds having the structure of formulas (Ia) or (Ib) or (IIa) or (IIb) as defined in claim 1 for the preparation of a vehicle for drug targeting of diagnostics or therapeutics.
